# EUROPEAN PATENT SPECIFICATION

(11) **EP 1 064 284 B1**
(45) Date of publication and mention of the grant of the patent: **27.03.2002**
(21) Application number: 99939844.9
(22) Date of filing: 17.03.1999
(51) Int. Cl.: C07D 471/04, C07D 221/04, A61K 31/435

(54) **HYPNOTIC BETA-CARBOLINE DERIVATIVES, PROCESS FOR THEIR PREPARATION AND THEIR USE AS MEDICINAL PRODUCTS**
HYPNOTISCHE BETA-CARBOLIN DERIVATE VERFAHREN ZU IHRER HERSTELLUNG UND IHRE VERWENDUNG ALS ARZNEIMITTEL
DERIVES DE BETA-CARBOLINE HYPNOTIQUE, PROCEDE POUR LEUR PREPARATION ET LEUR UTILISATION EN TANT QUE PRODUITS MEDICAUX

(30) Priority: 17.03.1998 US 42990
(43) Date of publication of application: 03.01.2001
(73) Proprietor: MACEF, 33000 Bordeaux (FR); LABORATOIRES BESINS INTERNATIONAL, 75003 Paris (FR)
(72) Inventor: FOURTILLAN, Jean-Bernard, F-86440 Migne-Auxances (FR); FOURTILLAN, Marianne, F-86440 Migne-Auxances (FR); JACQUESY, Jean-Claude, F-86180 Buxerolles (FR); JOUANNETAUD, Marie-Paule, F-86000 Poitiers (FR); VIOLEAU, Bruno, F-86370 Marcay (FR); KARAM, Omar, F-86280 Saint-Benoit (FR)
(74) Representative: Warcoin, Jacques
(86) International application number: IB9900494
(87) International publication number: WO9947521

(56) References cited:
- WO-A-96/08490
- US-A- 5 283 343
- H. HAMMER ET. AL.: "Dreiringreaktionen in der Indolreihe" CHEMISCHE BERICHTE, vol. 112, 1979, pages 1889-1901, XP002108405
- E. BÖSLING ET. AL.: "Stereoselektive Totalsynthese von Eburamonin, eburnamin und Eburnamenin." CHEMISCHE BERICHTE, vol. 112, 1979, pages 1902-12, XP002108406
- G. MASSIOT ET. AL.: "Synthesis in the indole series. VIII. A novel approach to indoloquinolizidines through alkylation-cyclisation of an enamine derived from tryptamine." TETRAHEDRON LETTERS, vol. 23, no. 2, 1982, pages 177-180, XP002108407
- H. TAKAYAMA ET. AL.: "On the Indole Alkaloid, Nauclefidine; Structure Revision, Synthesis, and a Biomimetic Transformation from the Vincoside Lactam. " TETRAHEDRON LETTERS, vol. 35, no. 47, 1994, pages 8813-6, XP002108408
- G. KALAUS ET. AL.: "Synthesis of Vinca Alkaloids and related compounds, XIII. Synthesis Starting from 2-(Ethoxycarbonyl)tryptamine." CHEMISCHE BERICHTE, vol. 114, 1981, pages 1476-83, XP002108409
- P. BENOVSKY ET. AL.: "Aza-Annulation as a Versatile Approach to the Synthesis of Non-Benzodiazepine Compounds for the Treatment of Sleep Disorders." TETRAHEDRON LETTERS, vol. 38, no. 49, 1997, pages 8475-8, XP002108410
- J-Y LARONZE ET. AL.: "Synthèses en série indolique V. Sur la réactivité de chloroindolénines dérivés d'oxo-4 indolo[2,3a]quinolizidines." BULLETIN OF THE CHEMICAL SOCIETY OF FRANCE, vol. 1977, 1977, pages 1215-20, XP002108411
- J. HARLEY-MASON ET. AL.: "A Simple Total Synthesis of Aspidospermidine" JOURNAL OF THE CHEMICAL SOCIETY, CHEMICAL COMMUNICATIONS, vol. 1967, 1967, pages 915-6, XP002108412
- M. INCZE ET. AL.: "Synthesis of Trifluoro-apovincaminic Acid Ester" ARCHIVE DER PHARMAZIE, vol. 323, 1990, pages 331-3, XP002108413 WEINHEIM; DE
- M. IHARA ET. AL.: "Novel synthesis of heterocyclic compounds having angular nitrogen atom by intramolecular Diels Alder reaction of 1-azadienes" HETEROCYCLES, vol. 23, no. 1, 1985, page 221 XP002108414
- M. NODE ET. AL.: "Chiral Total Synthesis of Indole Alkaloids of the Aspidisperma and Hunteria Types." JOURNAL OF ORGANIC CHEMISTRY, vol. 55, 1990, pages 517-21, XP002108415
- M. IHARA ET. AL.: "Stereocontrolled Synthesis of Indolo[2,3a]quinolizines by Intramolecular Double Michael Reaction: Proof for Stepwise Mechanism." JOURNAL OF THE CHEMICAL SOCIETY, CHEMICAL COMMUNICATIONS, vol. 1995, 1995, pages 2085-6, XP002108416
- B. DANIELI ET. AL.: "A One-Pot Syntheis of 2,6,7,12,Tetrahydroindolo[2,3a] quinolizin-4(3H)ones" SYNTHESIS , no. 4, 1984, pages 353-6, XP002108417
- H. HAMMER ET. AL.: "Aza- and oxa-eburnamonine (Reactions with indole derivatives, Part XLVII). " TETRAHEDRON, vol. 37, no. 21, 1981, pages 3609-13, XP002108418
- A. R. STOIT ET. AL.: "Models of Folate Cofactors. 20. An Approach to Deethyleburnamonine " TETRAHEDRON, vol. 45, no. 3, 1989, pages 849-54, XP002108419
- G. KALAUS ET. AL.: "Synthesis of Vinca Alkaloids and Related Compounds . 8. Unusual Alkylation of an Enamine." JOURNAL OF ORGANIC CHEMISTRY, vol. 43, no. 26, 22 December 1978, pages 5017-8, XP002108420
- S. B. MANDAL ET. AL: "Reduction of Lactams and Thiolactams by Sodium Borohydride: Application in the Synthesis of Some Alkaloids. " JOURNAL OF ORGANIC CHEMISTRY, vol. 53, no. 18, 2 September 1988, pages 4236-41, XP002108421
- B. DANIELI ET. AL.: "A new approach to Apovincamine." GAZZETTA CHIMICA ITALIANA, vol. 111, 1981, pages 257-66, XP002108422
- A. DANSCO ET. AL.: "Synthesis of Vinca Alkaloids and Related Compounds. XXIX. stereochemistry of 1,1-disubstituted indolo[2,3a]quinolizidines. " ACTA CHIMICA HUNGARICA, vol. 123, no. 3, 1986, pages 155-60, XP002108423

## Description

The present invention relates to novel β-carboline derivatives, to a process for their preparation and to their use as medicinal products.

Melatonin, N-acetyl-5-methoxytryptamine, is a hormone from the pineal gland, isolated by Lerner & al. (J. Am. chem. Soc., 80, 1958, 2587), which has formed the subject of numerous studies for its circadian activity, in the rhythm of sleep, for its effects on the production of testosterone, for its activity on the hypothalamus and in psychiatric disorders.

It has thus been envisaged to employ melatonin and analogues thereof especially for the treatment of depression and psychiatric disorders, in particular stress, anxiety, depression, insomnia, schizophrenia, psychoses and epilepsy, and also for the treatment of sleeping disorders associated with travelling ("jet lag"), neurodegenerative diseases of the central nervous system such as Parkinson's disease or Alzheimer's disease, for the treatment of cancers or, alternatively, as a contraceptive or as an analgesic.

However, the direct use of melatonin in vivo has not proved to be very satisfactory, on account of the facts that the first passage through the liver extracts more than 90 % of the active principle and that hypnotic activity of melatonin has not been clearly demonstrated.

Various melatonin analogues have been described, demonstrating two research routes which relate either to melatonin substituents (WO-A-89/01472, US-A-5 283 343, US-A-5 093 352 and WO-A-93/11761) or to the aromatic ring by replacing the indolyl group by a naphthyl group (FR-A-2 658 818, FR-A- 2 689 124).

The patent application WO 96 08490 describes novel melatonine agonist beta-carboline derivatives and analogues with a naphtalenic structure and their use for the treatment of disorders associated with melatonine deficiency, including anxiety and sleep disorders. However these compounds, which are not substituted in the 1-position, are totally hydrolyzed between pH 1 and pH 3, i.e. are not stable in acidic gastrict medium and therefore could not be administrated by oral route.

Benovsky et al. (*Tetrahedron Letters,* Vol. 38, Number 49, Pages 8475-8478, 1997), describes some hexahydro-indolo-quinolizin - 4 - ones for the treatment of sleep disorders. However these compounds do not have any methoxy substituent in the 9-position and no data was shown concerning their activity in the treatment of anxiety and sleep disorders.

We have demonstrated that melatonin does not exhibit any hypnotic activity ; but is the bioprecursor of acetylated metabolites which induce sleep.

The present patent application proposes a novel route for the development of novel carboline derivatives which are analogues of endogenous acetylated metabolites of melatonin.

The present invention relates to novel carboline derivatives of general formula I (I' and I'') :

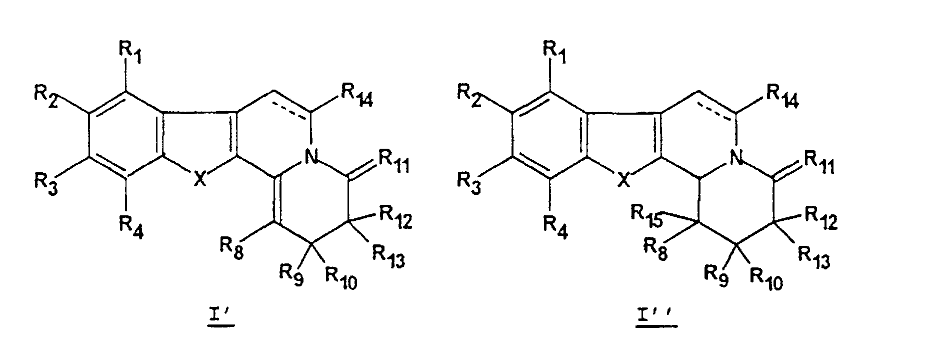

In which X represents a divalent radical of formula 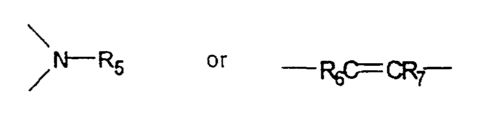
R₁, R₂, R₃, and R₄ represent, independently of each other, a hydrogen atom, a hydroxy radical, a lower alkyl, lower cycloalkyl, lower alkoxy, aryloxy, lower aralkyloxy, halo or nitro radical or an unsaturated aliphatic chain, formyl, lower alkylcarbonyl, lower alkylcarbonyloxy, halo(lower)alkylcarbonyl, halo(lower)alkylcarbonyloxy, halo(lower)alkyl, halo(lower)alkyloxy, lower alkoxycarbonyl, carboxyl, optionally substituted carboxamide, two adjacent radicals R₁, R₂, R₃ or R₄ being able to form together a 2,3-dihydropyranyl group which is possibly bearing an oxo group, R₁, R₂, R₃, and R₄ also represent, independently of each other, a perhalo(lower)alkyl, aryl, aralkyl, lower cycloalkoxy, polyhaloalkoxy, a thiol radical, lower alkylthio, lower cycloalkylthio, mono or polyhaloalkylthio, arylthio, aralkylthio, formate, a lower cycloalkylcarbonyloxy, arylcarbonyloxy, aralkylcarbonyloxy, lower dialkylaminoalkyl, aminoalkyl, lower alkylaminoalkyl, arylaminoalkyl, diarylaminoalkyl, arylalkylaminoalkyl, amino, alkylamino, dialkylamino, arylamino, diarylamino, aralkylamino, diaralkylamino, formamide, a lower alkylcarbonylamino, a lower cycloalkylcarbonylamino, halo(lower)-alkylcarbonylamino, polyhalo(lower)alkylcarbonylamino, arylcarbonylamino, lower aralkylcarbonylamino, a lower cycloalkylcarbonyl, polyhalo(lower)alkylcarbonyl, arylcarbonyl, aralkylcarbonyl, lower cycloalkoxycarbonyl, haloalkoxycarbonyl, aryloxycarbonyl, aralkyloxycarbonyl, a lower alkylsulfonyl, a lower cycloalkylsulfonyl, halo(lower)alkylsulfonyl, perhalo(lower) alkylsulfonyl, arylsulfonyl, aralkylsulfonyl, cyano radical.
R₅ represents a hydrogen atom, a lower alkyl, cycloalkyl, aryl, lower aralkyl, lower alkoxy, a lower alkylcarbonyl, halo(lower)alkylcarbonyl, lower alkyloxycarbonyl, amino, alkylamino, dialkylamino, alkylarylamino, diarylamino, halo(lower)alkylsulfonyl, alkylsulfonyl or arylsulfonyl radical,
R₅ also represents a halo(lower)alkyl, perhalo (lower)alkyl, a hydroxy radical, lower cycloalkoxy, aryloxy, aralkyloxy, a thiol radical, lower alkylthio, lower cycloalkylthio, arylthio, aralkylthio, dialkylaminoalkyl, arylamino, aralkylamino, diaralkylamino, optionally substitued carboxamide, formamide, formyl, a lower cycloalkylcarbonyl, perhalo(lower)alkylcarbonyl, arylcarbonyl, aralkylcarbonyl, lower cycloalkoxycarbonyl, haloalkoxycarbonyl, aryloxy-carbonyl, aralkyloxycarbonyl, a lower cycloalkylsulfonyl, perhalo(lower)alkylsulfonyl, aralkylsulfonyl, cyano radical.
R₆, R₇, R₉, R₁₀, R₁₂, R₁₃, R₁₄ and R₁₅ represent, independently of each other, a hydrogen atom, a lower alkyl, a lower cycloalkyl, halo(lower)alkyl, perhalo(lower)alkyl, aryl, aralkyl, lower alkoxy, lower cycloalkoxy, mono or polyhaloalkoxy, aryloxy, aralkyloxy, hydroxyalkyl, alkyloxyalkyl, lower alkylthio, lower cycloalkylthio, mono or polyhaloalkylthio, arylthio, aralkylthio, formate, lower alkylcarbonyloxy, a lower cycloalkylcarbonyloxy, halo-(lower)alkylcarbonyloxy, arylcarbonyloxy, aralkylcarbonyloxy, dialkylaminoalkyl, dialkylamino, diarylamino, diaralkylamino, optionally substitued carboxamide, formamide, a lower alkylcarbonylamino, a lower cycloalkylcarbonylamino, halo(lower)alkylcarbonylamino, perhalo-(lower)alkylcarbonylamino, lower arylcarbonylamino, lower aralkylcarbonylamino, formyl, a lower alkylcarbonyl, a lower cycloalkylcarbonyl, halo(lower)alkylcarbonyl, perhalo-(lower)alkylcarbonyl, arylcarbonyl, aralkylcarbonyl, carboxyl, lower alkoxycarbonyl, lower cycloalkoxycarbonyl, haloalkoxycarbonyl, aryloxycarbonyl, aralkyloxycarbonyl, a lower alkylsulfonyl, a lower cycloalkylsulfonyl, halo(lower)alkylsulfonyl, perhalo(lower) alkylsulfonyl, arylsulfonyl, aralkylsulfonyl, halo, cyano or nitro radical.
R₈ represents a lower alkyl, a lower cycloalkyl, hydroxyalkyl, alkyloxyalkyl, halo(lower)alkyl, perhalo-(lower)alkyl, aryl, lower-alkyl aryl, halo aryl, loweralkoxy aryl, nitro aryl, amino aryl, di(lower)alkylamino aryl, pyridyl, aralkyl, lower alkoxy, lower cycloalkoxy, mono or polyhaloalkoxy, aryloxy, aralkyloxy, lower alkylthio, lower cycloalkylthio, mono or polyhaloalkylthio, arylthio, aralkylthio, lower alkylcarbonyloxy, a lower cyclo-alkylcarbonyloxy, halo(lower)alkylcarbonyloxy, arylcarbonyloxy, aralkylcarbonyloxy, dialkylaminoalkyl, dialkylamino, diarylamino, diaralkylamino, optionally substitued carboxamide, formamide, a lower alkylcarbonylamino, a lower cycloalkylcarbonylamino, halo(lower)-alkylcarbonylamino, polyhalo(lower)alkylcarbonylamino, arylcarbonylamino, lower aralkyl-carbonylamino, formyl, a lower alkylcarbonyl, a lower cyclo-alkylcarbonyl, halo(lower)-alkylcarbonyl, perhalo(lower)alkylcarbonyl, aryl-carbonyl, aralkylcarbonyl, carboxyl, lower alkoxycarbonyl, lower cycloalkoxycarbonyl, haloalkoxycarbonyl, aryloxy-carbonyl, aralkyloxycarbonyl, a lower alkylsulfonyl, a lower cycloalkylsulfonyl, halo(lower) alkylsulfonyl, perhalo-(lower)alkylsulfonyl, arylsulfonyl, aralkylsulfonyl, halo, cyano or nitro radical.
R₆, R₇, R₈, R₉, R₁₀, R₁₂, R₁₃ and R₁₅ can represent, but not simultaneously R₈ and R₁₅, R₉ and R₁₀, R₁₂ and R₁₃ a hydroxyl radical, a thiol radical, amino, alkylamino, arylamino, aralkylamino, alkoxy, lower cycloalkoxy, mono or polyhaloalkoxy, aryloxy, aralkyloxy, lower alkylthio, lower cycloalkylthio, mono or polyhalogenoalkylthio, arylthio, aralkylthio, alkylamino, arylamino.

Furthermore 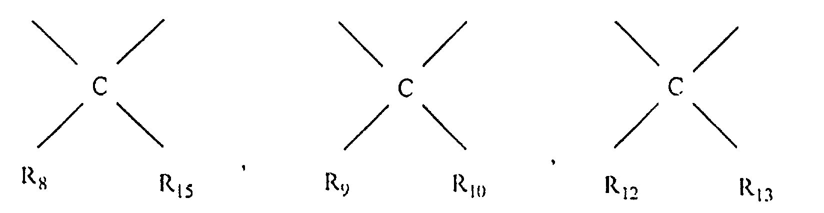 can also represent a carbonyl group C=O, a thiocarbonyl group C=S, a radical C=N-R₁₆, or a radical 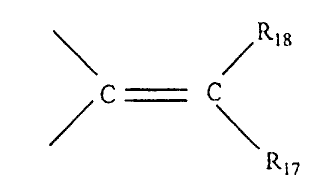
R₁₁ represents an oxygen atom, a sulfur,
R₁₆ represents a hydrogen atom, a lower alkyl, a lower cycloalkyl, aryl, aralkyl, a hydroxy radical, lower alkoxy, alkylcarbonyl, arylcarbonyl, aralkylcarbonyl, amino, alkylamino, dialkylamino, arylamino, diarylamino, aralkylamino, alkylcarbonylamino, arylcarbonylamino.
R₁₇ and R₁₈ represent independtly of each other a hydrogen atom, a lower alkyl, a lower cycloalkyl, aryl, aralkyl, lower alkoxy, lower cycloalkoxy, aryloxy, aralkyloxy, lower alkylthio, arylthio, aralkyltio, lower alkylcarbonyloxy, lower cycloalkylcarbonyloxy, arylcarbonyloxy, aralkyl-carbonyloxy, dialkylamino, arylalkylamino, diarylamino, optionnaly substitued carboxamide, formamide, lower alkylcarbonylamino, lower cycloalkylcarbonylamino, aryl-carbonylamino, formyl, lower alkylcarbonyl, lower cycloalkylcarbonyl, arylcarbonyl, aralkylcarbonyl, carboxyl, lower alkoxycarbonyl, aryloxycarbonyl, aralkyloxycarbonyl, lower alkylsulfonyl, arylsulfonyl.

It is possible for R₁-R₂, R₂-R₃, and R₃-R₄, to form part of another aromatic or non-aromatic ring with or without a hetero atom and optionally bearing a carbonyl or thiocarbonyl group.

Compounds, according to the present invention, may contain one to three assymetric centers, such compounds will exist as optical isomers (enantiomers).

The present invention concerns the racemic mixtures thereof, the pure enantiomers thereof or the mixtures thereof in all proportions, and the therapeutically acceptable salts thereof.

More particularly, the carbon atom bearing the R₈ group may be an assymetric center, the present invention includes all such enantiomers and mixtures.

The expressions lower alkyl, lower alkoxy or perhalo (lower) alkyl are generally understood to refer to radicals whose alkyl residue comprises between 1 and 6 carbon atoms.

These are preferably linear or branched C₁-C₄ alkyl residues chosen more particularly from methyl, ethyl, n-propyl, i-propyl, n-butyl, i-butyl and t-butyl groups. The expression unsaturated aliphatic chain is preferably understood to refer to an unsaturated C₂-C₆ hydrocarbon chain.

The term aryl generally denotes aromatic and heteroaromatic groups, in particular aryls chosen from phenyl, thienyl, furanyl, pyridyl and naphthyl groups. The aryl radicals may also be substituted with one or more groups chosen in particular from a hydrogen atom, a lower alkyl, a lower cycloalkyl, halo(lower)alkyl, perhalo-(lower)alkyl, aryl, aralkyl, a hydroxy radical, lower alkoxy, lower cycloalkoxy, mono or polyhaloalkoxy, aryloxy, aralkyloxy, a thiol radical, lower alkylthio, lower cycloalkylthio, mono or polyhaloalkylthio, arylthio, aralkylthio, formate, lower alkylcarbonyloxy, a lower cycloalkylcarbonyloxy, halo(lower)alkylcarbonyloxy, arylcarbonyloxy, aralkylcarbonyloxy, dialkylaminoalkyl, amino, alkylamino, dialkylamino, arylamino, diarylamino, aralkylamino, diaralkylamino, optionally substitued carboxamide, formamide, a lower alkylcarbonylamino, a lower cycloalkylcarbonylamino, halo(lower)alkylcarbonylamino, perhalo-(lower)alkylcarbonylamino, lower arylcarbonylamino, lower aralkylcarbonylamino, formyl, a lower alkylcarbonyl, a lower cycloalkylcarbonyl, halo(lower)alkylcarbonyl, perhalo-(lower)alkylcarbonyl, arylcarbonyl, aralkylcarbonyl, carboxyl, lower alkoxycarbonyl, lower cycloalkoxycarbonyl, haloalkoxycarbonyl, aryloxycarbonyl, aralkyloxycarbonyl, a lower alkylsulfonyl, a lower cycloalkylsulfonyl, halo(lower)alkylsulfonyl, perhalo(lower) alkylsulfonyl, arylsulfonyl, aralkylsulfonyl, halo, cyano or nitro radicals.

The expression lower aralkyl will be understood to refer to the combination of a lower alkyl and an aryl as defined above. This will preferably be the benzyl radical, which is optionally substituted.

The halo radicals are preferably chosen from fluorine, chlorine, bromine and iodine atoms.

The perhalo radicals are preferably perfluoro radicals.

When R₁-R₂, R₂-R₃ and R₃-R₄ form part of another aromatic ring, with or without a hetero atom, this is preferably another benzene ring, which is optionally substituted, or a pyridyl ring, which is optionally substituted.

When R₁-R₂, R₂-R₃ and R₃-R₄ form part of another nonaromatic ring, they preferably form together a divalent radical of formula -O-(CH₂)ₘ-, m being equal to 2 or 3, which is optionally substituted, or a divalent radical of formula -O-(CH₂)ₚ-O-, p being equal to 1 or 2, which is optionally substituted. When the derivatives comprise at least one asymmetric carbon, the present invention relates to the corresponding racemic mixtures, as well as to the pure enantiomers thereof or the mixtures thereof in all proportions.

The therapeutically acceptable salts of the derivatives according to the invention are the usual organic or inorganic salts of the art, in particular the hydrochlorides, the tosylates, the mesylates and the citrates, as well as solvates such as the hydrates or hemihydrates of the compounds of general formula I.

By comparison with the derivatives described in WO 96/08490, the derivatives according to the invention are characterised in that they contain a R₈ group which is different from an hydrogen atom, which increases dramatically the stability of such compounds in gastric acidic medium and allows oral administration to be used.

Without being bind to any theory, it has now been found that the group responsible of the hypnotic activity of the compounds according to the present invention is the ene-amide or dihydroene-amide group. This ene-amide or dihydroene-amide group is the group constituted by the Cₐ-N-C_{b} in formula I.

Derivatives according to the present invention and being of particular interest are these wherein :
R₁₁ represents preferably an oxygen atom or sulfur atom.
R₂ represents a hydroxy or lower alkoxy radical, preferably R₂ represents a methoxy radical.
R₁₄ represents a hydrogen atom or a lower alkyloxycarbonyl group.

The present invention relates more particularly to the derivatives of general formula Ia (Ia' and Ia").:

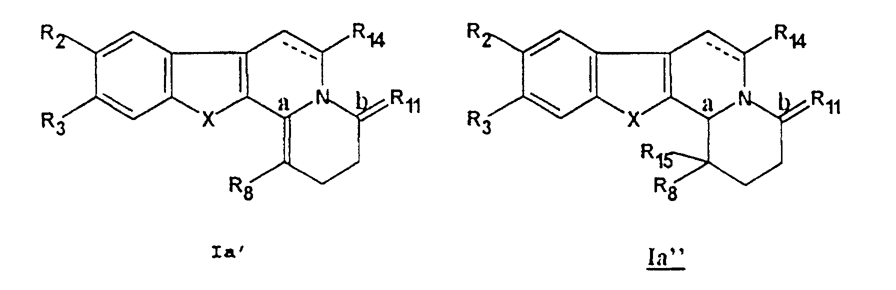

When Ia represents Ia',
R₈ represents an alkyl radical, optionally substitued by alkyl, halide, amino, alkyloxy groups, alkyloxycarbonyl, an aryl radical optionally substitued by alkyl, halide, nitro, dialkylamino, alkyloxy group, alkyloxycarbonyl, alkylamino, this aryl radical being e.g. a phenyl group, a pyridyl group

When Ia represents Ia'',
If R₁₅ is an hydrogen atom, R₈ represents preferably an ethyl, hexyl, isopropyl radical, a phenyl, fluorophenyl, methoxyphenyl, aminophenyl, dimethylaminophenyl, nitrophenyl, tolyl radical, an ethoxycarbonyl radical, a pyridyl radical.

If R₁₅ is different of an hydrogen atom, R₈ and R₁₅, identical, represent lower alkyl radicals or aryl radicals optionally substitued by alkyl, halide, amino, alkyloxy group.

X represents preferably a divalent radical of formula 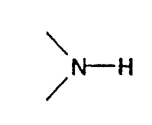 or

―HC=CH―

(R₅ = R₆ =R₇ = H)
R₁₁ represents preferably an oxygen atom or sulfur atom.
R₂ represents a hydroxyl or lower alkoxy radical and preferably a methoxy radical.
R₃ represents an hydrogen atom or an alkyl radical and preferably a methyl radical.

The present invention also relates to the process for the preparation of the derivatives of general formula I as defined above.

In the particular of derivatives of general formula Ib.

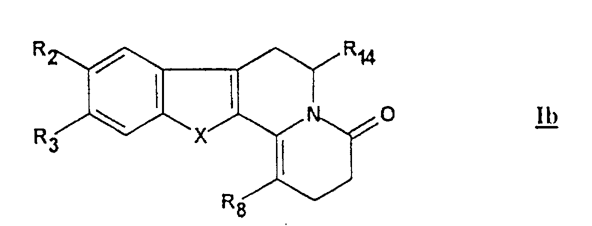

For which R₂, R₃, R₈ R₁₄ and X are defined above.

The derivative of formula Ib may be obtained directly by reacting the compound of general formula IIa.

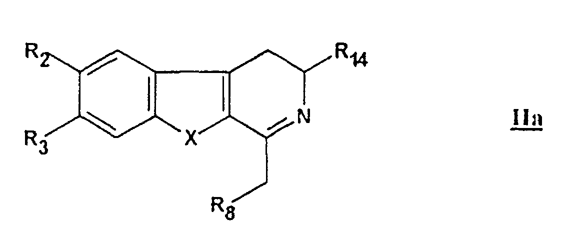

For which R₂, R₃, R₈, R₁₄ and X are defined above, with a carboxylic acid (such as acrylic acid) in the presence or not of diphenylphosphorylazide or with acrylonitrile.

In order to obtain the derivatives of general formula IIa, a Bischler-Napieralski reaction is carried out by reacting the compounds of general formula IIIa.

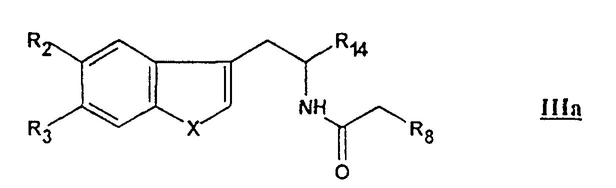

In which R₂, R₃, R₈, R₁₄ and X are defined above, with phosphorus pentoxide (P₂O₅) or with phosphorus oxychloride (POCl₃) in a suitable solvent, for example toluene, xylene, dichloromethane.

These derivatives IIa may also be prepared by permanganic oxidation of the derivatives of general formula IIb.

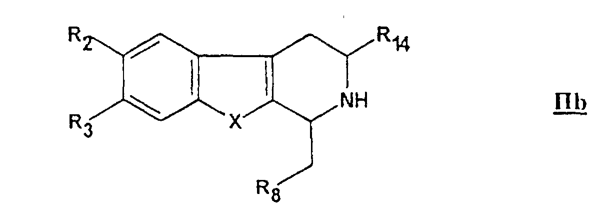

Wherein R₂, R₃, R₈ and R₁₄ are defined above.

In order to obtain the derivatives of general formula IIb, a Pictet-Spengler reaction is carried out by reacting the derivatives of general formula IIIb.

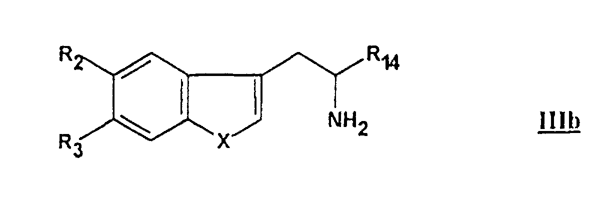

For which R₂, R₃, R₁₄ and X are defined above, with compounds of formula R₈-CH₂-CHO or chemical equivalents thereof such as a ketal, an enol ether, an enol ester or a nitrile of formula R₈-CH₂-CN under reducing conditions, R₈ being defined above.

The derivatives of formula IIIa may be obtained by carrying out an acylation by an acylating agent (acid chloride, acid anhydride, ester) on the derivatives of formula IIIb for which R₂, R₃, R₁₄ and X are defined above.

Also, when X=NR₅ the derivatives of formula IIIa may be obtained by Fischer reaction with the appropriate substituted phenylhydrazine of general formula IV and the suitable aldehyde or masked aldehyde such ketal of general formula V.

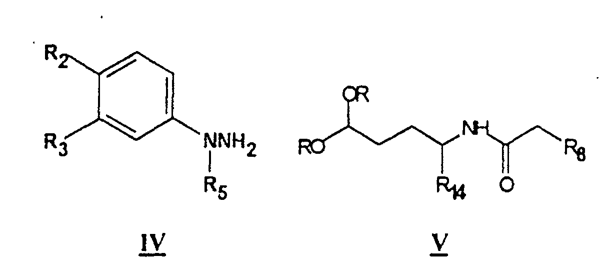

For which R₂, R₃, R₈, R₁₄ are defined above
R₅ represents an hydrogen radical or a lower alkyl

In the particular case of derivatives of general formula Ic

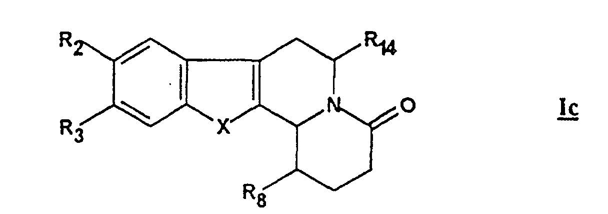

For which R₂, R₃, R₈, R₁₄ and X are defined above, the derivative of formula Ic
may be obtained directly by catalytic reduction by hydrogen with Palladium on Carbon of compound of general formula Ib.

In the particular case of derivatives of general formula Id

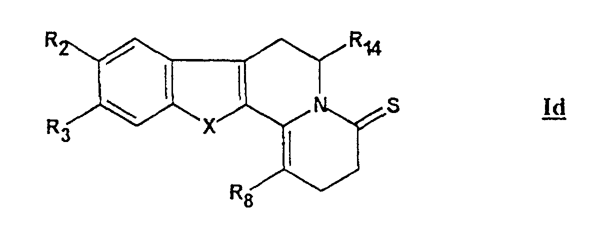

For which R₂, R₃, R₈, R₁₄ and X are defined above, the derivatives of general formula Id may be obtained directly by reaction of the Lawesson's reagent or by P₂S₄ on the compounds of general formula Ib.

In the particular case of derivatives of general formula Ie 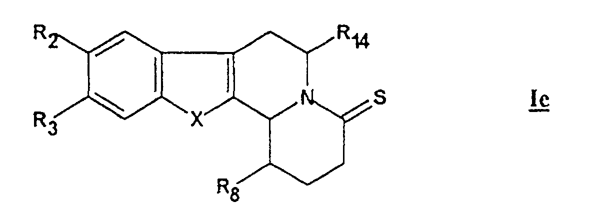 for which R₂, R₃, R₈, R₁₄ and X are defined above, the derivative of formula Ie may be obtained directly by reaction of the Lawesson's reagent or by P₂S₄ on the compound of general formula Ic.

In the particular case of derivatives of general formula If 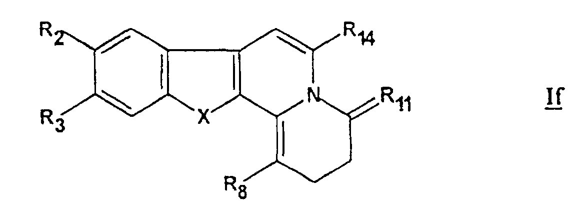 for which R₂, R₃, R₈, R₁₁, R₁₄ and X are defined above, the derivative of formula If may be obtained directly with an oxidant (such O₂ in alkaline medium) on the compound of general formula Ib or Id.

The derivatives according to formula Ia wherein R₈ and R₁₅ are identical may be obtained by carrying out a reaction on the derivatives of general formula IIIb 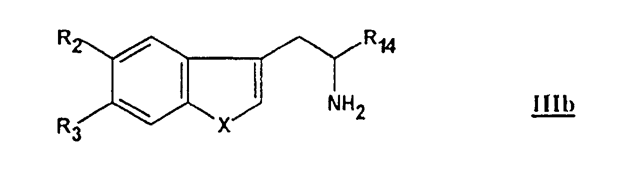 with compounds of formula EtO-CO-C(R₈) (R₁₅)-COH or chemical equivalents thereof under reducing conditions

The derivatives according to the invention may be used as medical product for the treatment of diseases associated with disorders of melatonin activity. The derivatives according to the invention present myorelaxing properties and they also may be use for the treatment of depression and psychiatric disorders, in particular stress, anxiety, depression, insomnia, schizophrenia, psychoses and epilepsy, and also for the treatment of sleeping disorders associated with travelling ("jet lag"), neurodegenerative diseases of the central nervous system such as Parkinson's disease or Alzheimer's disease, for the treatment of cancers or, alternatively, as a contraceptive or as an analgesic. They present hypnotic and sedative effects.

### STABILITY IN ACIDIC MEDIUM AND PHARMACOLOGICAL ACTIVITY

### 1.STABILITY IN ACIDIC MEDIUM AND ORAL BIOAVAILABILITY IN BEAGLE DOG

In order to study the influence of R₈ group on the stability of cycle A when comparing compounds EXAMPLE A and EXAMPLE B (R₈ = hydrogen atom) to compounds of Example 1, Example 4, Example 7, Example 8 and Example 11, 30 µM of each of these compounds were dissolved into 5 ml of pH=1 or pH=2 or pH=3 or pH=7 (reference) buffer. Solutions were stirred for 15 minutes at 37°C. Subsequent measurements of compounds amounts non hydrolyzed were measured using a high performance liquid chromatography method.

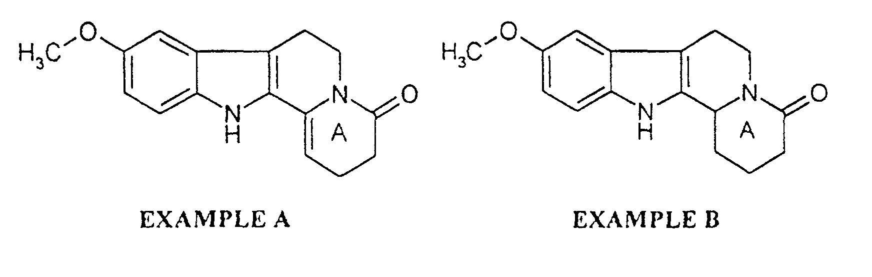

**Table I :**

| Stability in acidic medium and oral bioavailability in beagle dogs | | | | | |
|---|---|---|---|---|---|
| | **Hydrolysis rate (%)** | | | | **Absolute bioavailability oral/IV in Beagle dogs (fasting conditions) 1 < gastric pH < 2** |
| **Example n°** | **pH=1** | **pH=2** | **pH=3** | **pH=7 (reference)** | |
| A | 100 | 100 | 100 | 0 | <1% |
| 1 | 79.5 | 14.5 | 8.7 | 0 | >35% |
| 7 | 17.0 | 13.0 | 5.4 | 0 | N.D. |
| 4 | 0 | 0 | 0 | 0 | >20% |
| 11 | 26.0 | 27.0 | 30.0 | 0 | N.D. |
| B | 95 | 95 | 90 | 0 | <5% |
| 8 | 0 | 0 | 0 | 0 | 100% |
| N.D. : Not determined | | | | | |

Results are presented in Table I as percentage of compounds hydrolyzed, as shown in Table I, when compared to compounds EXAMPLE A and EXAMPLE B which are totally hydrolyzed between pH=1 and pH=3.
Compounds n°1, 4, 7, 8 and 11 are quite stable from pH=2, since less than 27 per cent of these compounds are hydrolysed at pH≥2.

In conclusion, compounds wherein the R₈ group (in general formula I) is different from a hydrogen atom do not undergo a total hydrolysis of cycle A. The stability in acidic gastric medium of compounds such as examples 1, 4, 8 allows their administration by oral route. Pharmacokinetic studies of these compounds (Table I) have been carried out in beagle dogs ; compounds 1, 4, 8 exhibit absolute bioavailability of oral route relative to intravenous route respectively higher than 35% (compound 1), 20% (compound 4) and equal to 100% (compound 8), since EXAMPLE A and EXAMPLE B absolute bioavailability are lower than 1% (EXAMPLE A) and 5% (EXAMPLE B).

Compounds n°1 (see Table II), as well as compounds n°4 and 7 (see Table III) exhibit significant hypnotic effect in Beagle dogs when given orally.

### 2. HYPNOTIC ACTIVITY IN BEAGLE DOGS : EFFECTS ON WAKEFULNESS / SLEEP STATES IN BEAGLE DOGS

Beagle dogs are kept in a metal cage, connected to 2 Schwartzer ED 24 Polygraph equipped with BRAINLAB® software, through a flexible cable.
Test and control products are administered via the oral or intravenous routes by means of gastric tubes. Records are scored in 30 seconds epochs for active wake, drowsy, slow wave sleep (light sleep + deep sleep) and REM sleep. These criteria are according to SHELTON et al.( in SHELTON J., NISHINO S., VAUGHT J., DEMENT W.C. and MIGNOT E. Comparative effects of modafinil and amphetamine on daytime sleepiness and cataplexy of narcoleptic dogs. Sleep 19(1) ; 29-38, 1996.), based upon frequency and amplitude patterns of EEG cortical tracing (fronto-frontal), EMG (upper neck muscles) and EOG (bilateral). Behaviour is also continuously monitored for each observation period of 120 minutes. Prior acclimatisation of the dogs take 5 days ; and, to mimic similar stress caused by gastric tubing, a mixture water/PEG (50/50 ; V/V) was administered orally together with intravenous boluses.
- Wake : includes all episodes with low voltage mixed frequency tracing in which the EMG is not inhibited. During wakefulness dogs stand, sit, or lay down, and eyes are open.
- Sleepiness : the drowsy state is scored when dogs lie quietly with eyes closed (50 p. cent or more per one epoch) and cortical EEG shows trains of relatively slow waves (4 - 7 Hz) without the development of sleep spindles. Synchronous waves at 4 - 7 Hz and 50 - 100 µV appear on a background of low voltage fast-wave activity.
   EMG is moderately decreased compared to wake stage.
- Light deep sleep = slow wave sleep (SWS) (light sleep + deep sleep) : canines are relaxed with prone posture. EEG patterns are of higher amplitude than in the previous stage and sleep spindles ( 10 - 14 Hz)
   and / or K complexes must be present in light sleep, as EOG show slow eye movements or no movement. Deep sleep is scored when slow delta waves (< 4 Hz) constitute 20 p. cent of an epoch or more during deep sleep.
- REM : rapid eye movement sleep is scored when dogs lay down, eyes closed with intermittent apparent fast muscle twitches. A low voltage mixed frequency EEG tracing is observed together with rapid eye movements and a drop in EMG activity.

Repartition and duration of wakefulness / sleep states in Beagle dogs have been measured after oral (and intravenous route for compoundof EXAMPLE 1) administrations of Placebo (vehicle = 10 ml of mixture ethanol / PEG 400 / water ; 10 / 40 /50 ; V/V/V) compounds of Example 1, Example 4 and Example 7.

Results are presented in Tables II and III as Durations and Latencies of each stage.

Compounds of Example 1, Example 4 and Example 7 exhibit a potent hypnotic activity inducing a sleep characterized by a high proportion of slow wave sleep. Regarding duration and latencies of wake, drowsy and SWS stages, these 3 compounds induce a significant hypnotic effect.

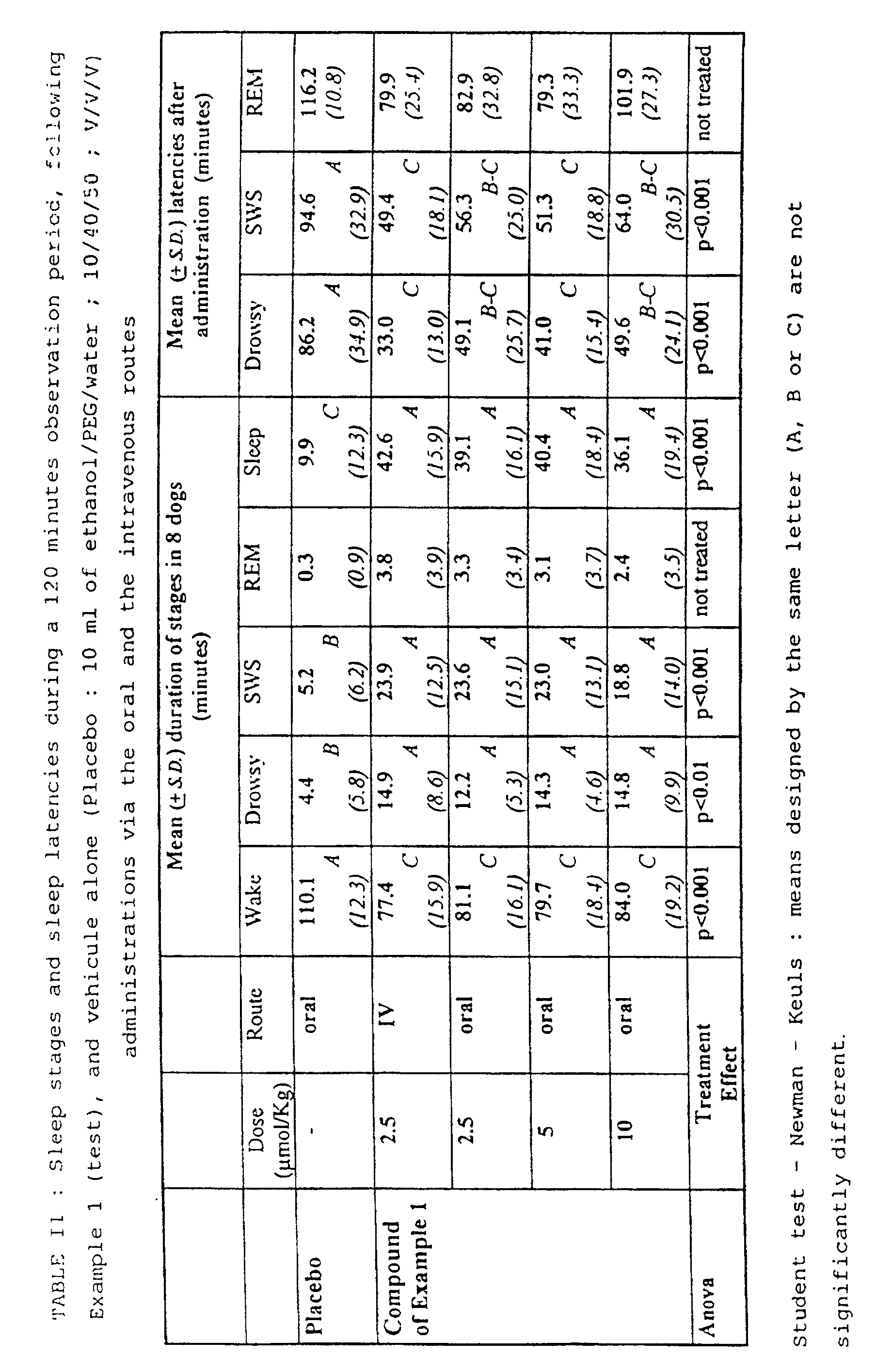

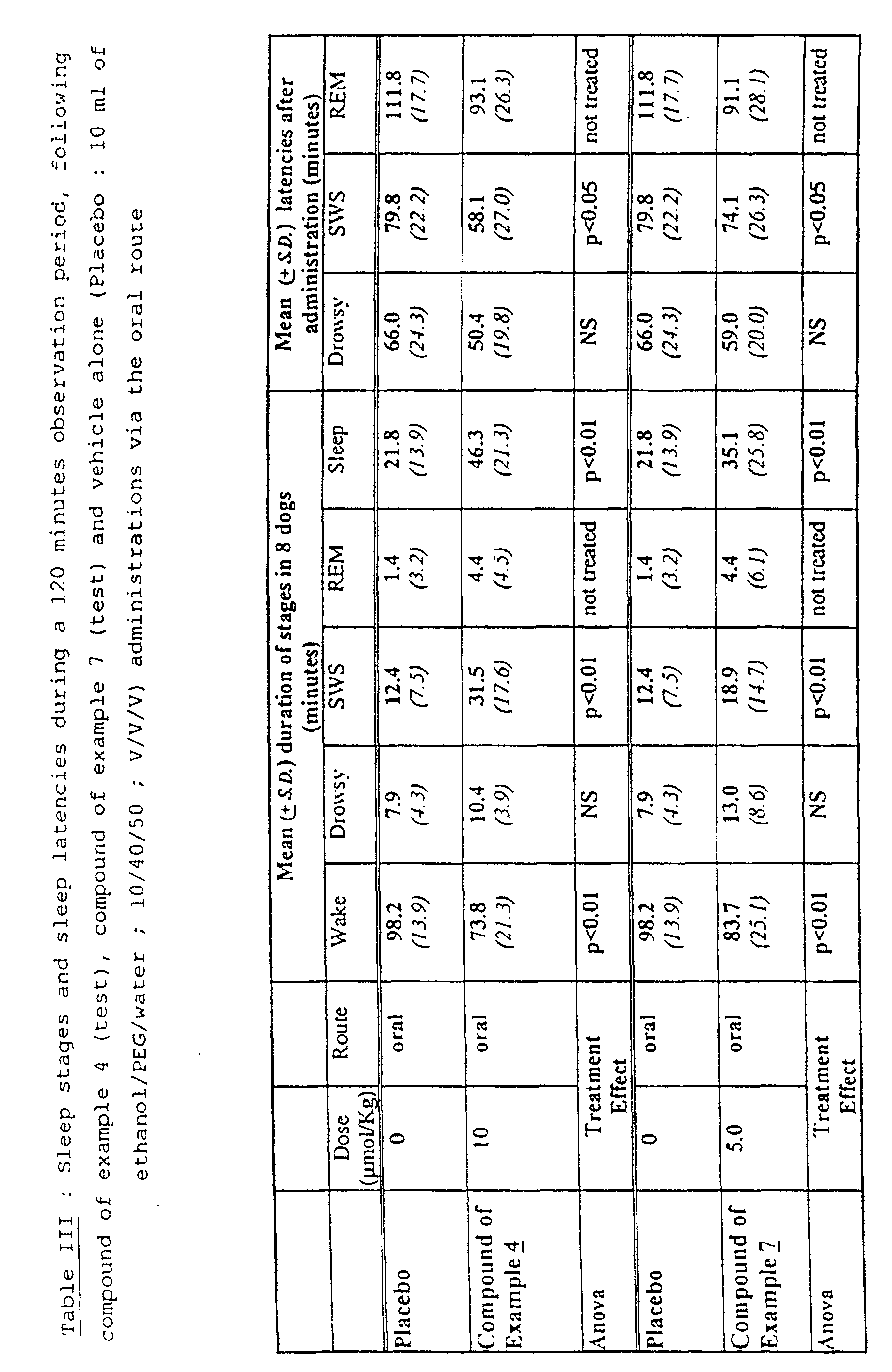

### 3. HYPNOTIC AND SEDATIVE EFFECTS IN CHICKS

The hypnotic and sedative effects of the derivatives, according to the invention, prepared above (the test results of which are given in Table IV below) were compared with those of 3 reference products, diazepam, pentobarbital sodium and melatonin, as well as with 2 psychostimulant compounds with hallucinogenic properties : 10-methoxyharmalan and harmaline, which are 3,4-dihydro-β-carbolines, in 10- to 14-day-old chicks of chair label JA657 strain. The animals are subjected to alternating programmes of lighting consisting of 12 h of darkness (20.00 h to 8.00 h) and 12 h of light (8.00 h to 20.00 h). The ambient temperature is 25°C during the first week of rearing of the chicks and 22°C from the second week onwards. During the day, the lighting is provided by a halogen lamp (300 W) placed 30 cm above the floor of the vivarium. During the tests, the live weights of the chicks ranged between 85 and 120 g. The tests are carried out between 14.00 and 15.00 h. The chicks are allotted, in groups of 3, in identical 30 cm x 50 cm x 30 cm vivariums. The test products are administered intramuscularly (IM) into the pectoralis major muscle, as a solution in a 25/50/25 (V/V/V) ethanol / PEG 400 / water mixture, at the rate of 0.2 ml of solution per 100 g of live weight. The doses administered for the test products (novel compounds of the invention and reference substances) ranged from 0.25 µmol to 2 µmol per 100 g of live weight. The placebo corresponds to 0.2 ml of the 25/50/25 (V/V/V) ethanol / PEG 400 / water mixture.

The solutions of the tests products in the 25/50/25 (V/V/V) ethanol / PEG 400 / water / mixture were prepared at the times of use by successive dilution of a stock solution, obtained from 2.5 to 20 µmol of accurately weighed product, to which were successively added 0.5 ml of pure ethanol and then 1 ml of PEG 400, agitated by ultrasound and then made up to 2 ml with 0.5 ml of distilled water for an injectable preparation. Table IV gives the results obtained after IM administration of doses of between 0.25 and 2 µmol of test products dissolved in 0.2 ml of the ethanol / PEG 400 / distilled water mixture (25/50/25 ; V/V/V), per 100 g of live weight. For each chick, the volume injected is adjusted, as a function of the actual live weight, to 0.2 ml per 100 g of live weight.

The parameters observed are the locomotor activity and state of consciousness of the chicks for 2 h, i.e. the equivalent of 6 theoretical awake/asleep cycles for a chick of this age. They are recorded by video camera for 90 minutes, the first 30 minutes being the time for adaptation to the device.

The hypnotic and sedative effects of the test products on the diurnal activity of 10- to 14-day-old chicks subjected to a program of permanent lighting from birth for 48 h, and then to alternate lighting program of 12 h of daylight (8.00 h - 20.00 h) and 12 h of darkness (20.00 h - 8.00 h) up to the test date, are given in Table I below. The tests are carried out during the day between 14.00 h and 15.00 h.

For each test product, several series of measurements were taken on batches of 3 animals, each value indicated being the average in each batch of 3 chicks. When the number of batches is greater than or equal to 2, the figures indicated are the average limit values observed.

**Table IV**

| **Compound** | **Dose (µmol/100 g)** | **Dose (mg/kg)** | **FAT (min.)** | **ST (min.)** | **Sedation time (min.)** |
|---|---|---|---|---|---|
| Placcbo | (24 batches) | | NA | 0 | 10 - 35 |
| Melatonin | 0.5 | 1.16 | NA | 0 | Not determined |
| | 1 (5 batches) | 2.32 | NA | 0 | 16-36 |
| | 2 (5 batches) | 4.64 | NA | 0 | 47 - 105 |
| Pentobarbital | 0.5 (3 batches) | 1.24 | NA | 0 | Not determined |
| | 1 | 2.48 | 13 | 36 | Not determined |
| Diazepam | 0.5 (4 batches) | 1.42 | 3 - 6 | 10 - 50 | Not determined |
| | 1 (10 batches) | 2.85 | 2 - 7 | 24 - 70 | 17 - 20 |
| | 2 (3 batches) | 5.69 | 2 - 5 | 81 - 100 | 14 - 15 |
| 10-methoxyharmalan | 1.4 | 3 | NA | 0 | 0 |
| Harmaline | 1.4 | 3 | NA | 0 | 0 |
| Example 1 | 0.25 | 0.74 | 10 | 16 | 15 |
| | 0.50 | 1.48 | 9 | 18 | 17 |
| | 1 (8 batches) | 2.96 | 6 - 14 | 28 - 101 | 5 - 20 |
| Example 3 | 0.75 | 2.32 | 12 | 23 | 1 |
| Example 4 | 0.25 | 0.86 | 10 | 9 | 17 |
| | 0.50 | 1.72 | 12 | 11 | 8 |
| | 1 (4 batches) | 3.44 | 13 - 15 | 13 - 27 | 22 - 32 |
| Example 5 | 0.75 | 2.44 | 12 | 10 | 10 |
| Example 6 | 1 | 3.24 | 8 | 102 | 5 |
| Example 7 | 0.5 | 1.56 | 8 | 28 | 10 |
| | 1 | 3.12 | 5 | 35 | 30 |
| Example 8 | 2 | 5.97 | 15 | 15 | 37 |
| Example 9 | 1 | 3.07 | 11 | 43 | 16 |
| Example 11 | 0.5 | 1.80 | 9 | 20 | 8 |
| | 1 | 3.60 | 8 | 25 | 24 |
| Example 13 | 1 | 3.62 | 15 | 29 | 23 |
| Example 14 | 1 | 3.74 | 10 | 15 | 29 |
| Example 16 | 1 | 3.82 | 6.5 | 15 | 42 |
| Example 17 | 1 | 3.89 | 11 | 7 | 38 |
| Example 18 | 1 | 3.58 | 5 | 16 | 58 |
| Example 19 | 1 | 3.46 | 2 | 35 | 33 |

Key :
- NA : not applicable, the animals remained conscious throughout the period of observation ;
- FAT : falling-asleep time, equal to the time required to pass from the state of active consciousness to a non-conscious state ;
- ST : sleep time, equal to the duration of the period of sleep from falling asleep to waking up ;
- Sedation time : after waking up, period of inactivity corresponding to stage 2 defined above.

In the conditions under which the test is carried out (times of administration, in the phase during which the animals receive light, between 14.00 h and 15.00 h) melatonin has no hypnotic activity.
By successively subjecting chicks to programmes of alternate and permanent lighting, we have demonstrated experimentally that melatonin has no direct hypnotic activity which is intrinsic to its structure. Its hypnotic activity depends on the activity of the enzyme N-acetyltransferase (NAT) in the pineal gland of the chick at the time of administration of the melatonin. The NAT enzyme is an acetylation enzyme. In the presence of the NAT enzyme in the pineal gland of the chick, the IM administration of melatonin induces a hypnotic effect of strong intensity (sleep time of between 250 and 300 minutes for a dose equal to 1 µmol of melatonin/100 g of live weight). Melatonin is thus the precursor of acetylated metabolites with direct hypnotic activity. Compounds of the invention are analogues of the hypnotic acetyl metabolites of melatonin.
In contrast with melatonin, all of the derivatives of the invention described above have direct hypnotic and sedative activities, which are independent of the time of administration, i.e. of the level of N-acetyltransferase enzyme in the CNS.
The results obtained show, for the derivatives according to the invention, a hypnotic effect which is higher than that of the reference products (pentobarbital, melatonin) and equivalent or even superior to that of diazepam.
The derivatives according to the invention are thus particularly advantageous for the treatment of sleep disorders and diseases associated with disorders of melatonin activity.

### EXAMPLES

### Example 1: ETCARBO7

Formula : C₁₈H₂₀N₂O₂ M = 296, 36 g.mol-1

### Structure :

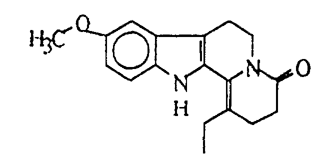

### 9-methoxy-1-ethyl-2,3,4,6,7,12-hexahydroindolo [2,3-a] quinolizin-4-one

### Preparation :

Paramethoxyphenylhydrazine sulfonate (5 g - 20,7 mmol) and N(4, 4 -diéthoxybutyl)butanamide ( 4,8 g - 20,7 mmol)are mixed in commercial THF (85 ml) in a 500 ml flask. The medium is then heated to the THF reflux and acetic acid (25 %) is added dropwise (35 ml). The limpid yellow mixture is stirred for 6 h at a temperature comprised between 80 and 85°C. After cooling, the reacting medium is transferred in a 2 litre Erlenmeyer flask and it is basified by addition of a sodium carbonate saturated solution (∼100 ml) pH >7. The organic phase is decanted, and the aqueous phase is twice extracted with ethyl acetate (2 X 100 ml). The organic phases are jointed and successively washed with a sodium carbonate saturated solution (70 ml) and with water(70 ml). The obtained organic phase is dried over MgSO4 and the solvent is evaporated under reduced pressure until crystals appear (∼5 ml ethyl acetate). After dilution with diethylic ether (50 ml) this solution is left overnight in the refrigerator. The crystals are obtained by a filtration, washed with diethylic ether and dried under vacuum. N1-(2-(5-methoxy-lH-3-indolyl)ethyl)butanamide (3,3 g - R= 61%) is then obtained.

A Bischler-Napieralski reaction on N1-(2-(5-methoxy-1H-3-indolyl)ethyl)butanamide leads to the 1-propyl-6-methoxy-3,4-dihydro-2-carboline.

### Method 1:

Acrylic acid (0.71 ml, 1.1 cq.) is added to a solution of 1-propyl-6-methoxy-3,4-dihydro-2-carboline (2.34 g) in DMF (20 ml). Diphenylphosphoryl azide (2.1 ml, 1.06 eq.) dissolved in DMF (3 ml) is then added dropwise, followed by triethylamine (2.85 ml, 2.1 eq.). After recrystallization from ethyl acetate, 9-methoxy-1-ethyl-2,3,4,6,7,12-hexahydroindolo[2,3-a]quinolizin-4-one is recovered (1.6 g, 56%).

### Method 2:

Acrylic acid (1 eq.) dissolved in xylene is added to a solution of 1-propyl-6-methoxy-3,4-dihydro-2-carboline in xylene. The reaction flask is equipped with a water separator and the medium is heated to reflux of the xylene for 24 h. The xylene is then distilled off under reduced pressure. The product is purified as above.
**NMR:** ^{**1**}**H** (CDCl₃) : 1.29 (t, 3H); 2.44 and 2.54 (2m, 6H) ; 2.86 (t, 2H); 3.86 (s, 3H); 4.08 (t, 2H); 6.87 (dd 2.4 and 8.7 Hz, 1H); 6.95 (d 2.4 Hz, 1H); 7.27 (d 8.7 Hz, 1H); 8.04 (broad s, 1H)
**Mass spectrum**:
m/z: 296(M+.), 281(100)

| | | |
|---|---|---|
| **Exact mass** | calculated | 296.1524 |
| | found | 296.1545 |

**Melting point**: 223°C

### Example 2 : HECARBO7

Formula : C₂₂H₂₈N₂O₂ M = 352, 47 g.mol-1

### Structure :

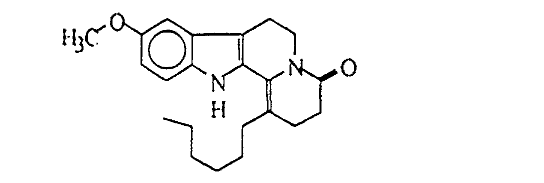

### 9-methoxy-1-hexyl-2,3,4,6,7,12-hexahydroindolo[2,3-a] quinolizin-4-one

### Preparation :

A Bischler-Napieralski reaction on N1-(2-(5-methoxy-1H-3-indolyl)ethyl)octanamide leads to the 1-heptyl-6-methoxy-3,4-dihydro-2-carboline.
Acrylic acid (1 eq.) dissolved in xylene is added to a solution of 1-heptyl-6-methoxy-3,4-dihydro-2-carboline in xylene. The reaction flask is equipped with a water separator and the medium is heated to reflux of the xylene for 48 h. The xylene is then distilled off under reduced pressure. The product is recrystallized from ethyl acetate.
**NMR:** ^{**1**}**H** (CDCl₃): 0.92 (t, 3H); 1.42 (m, 8H); 2.40 (t, 2H); 2.50 (m, 4H); 2.56 (t, 2H); 3.86 (s, 3H); 4.08 (t, 2H); 6.87 (dd 2.4 and 8.7 Hz, H); 6.94 (d 2.4 Hz, 1H); 7.23 (d 8.7 Hz, 1H); 8.04 (broad s, 1H)
**Mass spectrum**:
m/z: 352(M+.), 281(100)
**Melting point**: 140°C

### Example 3 : IPCARBO7 :

Formula : C₁₉H₂₂N₂O₂ M = 310,39 g.mol-1

### Structure :

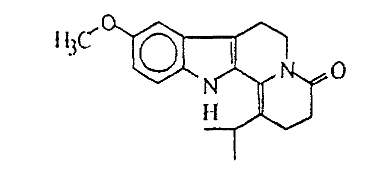

### 9-methoxy-1-isopropyl-2,3,4,6,7,12-hexahydroindolo[2,3-a] quinolizin-4-one

### Preparation :

A Bischler-Napieralski reaction on N1-(2-(5-methoxy-1H-3-indolyl)ethyl)-3-methylbutanamide leads to the 1-isobutyl-6-methoxy-3,4-dihydro-2-carboline.
Acrylic acid (1 eq.) dissolved in xylene is added to a solution of 1-isobutyl-6-methoxy-3,4-dihydro-2-carboline in xylene. The reaction flask is equipped with a water separator and the medium is heated to reflux of the xylene for 48 h. The xylene is then distilled off under reduced pressure. The product is recrystallized from ethyl acetate.
**NMR:** ^{**1**}**H** (CDCl₃) : 1.20 (d, 6H); 2.35 and 2.47 (2m, 4H); 2.87 (t, 2H); 3.38 (m, 1H); 3.86 (s, 3H); 4.06 (t, 2H); 6.87 (dd 2.4 and 9 Hz, 1H); 6.95 (d 2.4 Hz, 1H); 7.32 (d 9 Hz, 1H);
**Mass spectrum:**
m/z: 310(M+.), 295(100)
**Melting point:** 251-252°C

### Example 4 : PHCARBO7

Formula : C₂₂H₂₀N₂O₂ M = 344,41 g.mol⁻¹

### Structure :

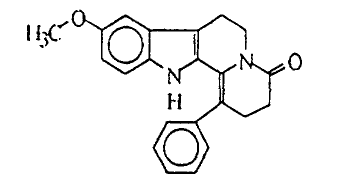

### 9-methoxy-1-phenyl-2,3,4,6,7,12-hexahydroindolo[2,3-a] quinolizin-4-one

### Preparation :

A Bischler-Napieralski reaction on N1-(2-(5-methoxy-1H-3-indolyl)ethyl)-2-phenylacetamide leads to the 1-benzyl-6-methoxy-3,4-dihydro-2-carboline.
Acrylic acid (0.75 ml, 1.1 eq.) is added to a solution of 1-benzyl-6-methoxy-3,4-dihydro-2-carboline (2.8 g) in DMF (20 ml). Diphenylphosphoryl azide (2.1 ml, 1.06 eq.) dissolved in DMF (3 ml) is then added dropwise, followed by triethylamine (2.85 ml, 2.1 eq.). After separation on silica gel (chloroform/methanol eluent), 9-methoxy-1-phenyl-2,3,4,6,7, 12-hexahydroindolo[2,3-a]quinolizin-4-one is recovered (1.6 g, 56 %).
**NMR:** ^{**1**}**H** (CDCl₃) : 2.71 (m, 4H); 2.91 (t, 2H); 3.83 (s, 3H); 4.20 (t, 2H); 6.76 (dd 2.4 and 8.7 Hz, 1H); 6.84 (d 8.7 Hz, 1H); 6.90 (d 2.4 Hz, 1H); 6.93 (broad s, 1H); 7.42 and 7.50
(m, 5H)
**Mass spectrum:**
m/z: 344(M+.)(100), 253
**Melting point:** 235°C

### Example 5 : CO2ETCARBO7

Formula : C₁₉H₂₀N₂O₄ M = 340,37 g.mol⁻¹

### Structure :

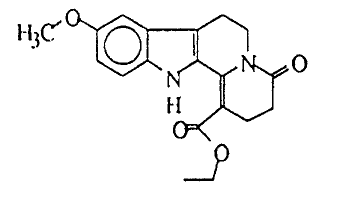

### 1-carbethoxy-9-methoxy-2,3,4,6,7,12-hexahydroindolo[2,3-a] quinolizin-4-one

### Preparation :

A Bischler-Napieralski reaction on ethyl 3-((2-(5-methoxy-1H-3-indolyl)ethyl)amino)-3-oxopropanoate leads to the corresponding carboline.
Sodium hydroxide solution (1N, 3 ml) is added to a solution of the carboline obtained (800 mg) in benzene (10 ml), followed by tetrabutylammonium hydrogen sulphate (0.1 eq.). Acryloyl chloride (0.27 ml) is then added at 0°C and the mixture is allowed to return to room temperature overnight. The product is separated on silica gel (chloroform/methanol) and 1-carbethoxy-9-methoxy-2,3,4,6,7,12-hexahydroindiolo[2,3-a]quinolizin-4-one is thus obtained.
**NMR:** ^{**1**}**H** (CDCl₃): 1.42 (t, 3H); 2.60 (t, 2H); 2.85 (t, 2H); 2.97 (t, 2H); 3.89 (s, 3H); 4.30 (t, 2H); 4.35 (q, 2H); 6.96 (d 2.1 Hz, 1H); 7.01 (dd 2.1 and 7.5 Hz, 1H); 7.35 (d 7.5 Hz, 1H) ;
**Mass spectrum:**
m/z: 340(M⁺), 294(100)
**Melting point:** 174-175°C

### Example 6 : 6ETETCARBO7

Formula : C₂₀H₂₄N₂O₂ M = 324,42 g.mol⁻¹

### Structure :

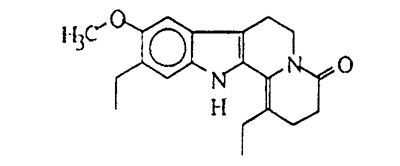

### 9-methoxy-1,10-diethyl-2,3,4,6,7,12-hexahydroindolo[2,3-a] quinolizin-4-one

### Preparation :

A Bischler-Napieralski reaction on N1-(2-(5-methoxy-6-ethyl-1H-3-indolyl)ethyl)butanamide leads to the 7-ethyl-1-propyl-6-methoxy-3,4-dihydro-2-carboline.
Acrylic acid (0.22 ml, 1.1 eq.) is added to a solution of 7-ethyl-1-propyl-6-methoxy-3,4-dihydro-2-carboline (764 mg) in DMF (20 ml),acrylic acid (0.22 ml, 1.1 eq)is added. Diphenylphosphoryl azide (1.06 eq.) dissolved in DMF (3 ml) is then added dropwise, followed by triethylamine (2.1 eq.). After separation on silica gel (chloroform/methanol eluent) 9-methoxy-1,10-diethyl-2,3,4,6,7,12-hexahydroindolo[2,3-a] quinolizin-4-one is recovered (28%).
**NMR:** ^{**1**}**H** (CDCl₃) : 1.26 (m, 6H); 2.41 and 2.58 (2m, 6H); 2.70 (q, 2H); 2.87 (t, 2H); 3.87 (s, 3H); 4.07 (t, 2H); 6.88 (1s, 1H); 7.19 (s, 1H); 8.33 (broad s, 1H).
**Mass spectrum:**
m/z: 324(M+.), 309(100)
**Melting point:** 204°C

### Example 7 : ETCARBO7S

Formula : C₁₈H₂₀N₂OS M = 312,42 g.mol⁻¹

### Structure :

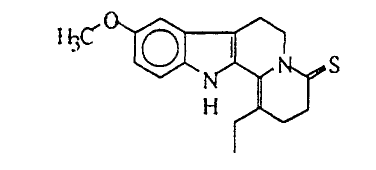

### 9-methoxy-1-ethyl-2,3,4,6,7,12-hexahydroindolo[2,3-a] quinolizine-4-thione

### Preparation:

Lawesson's reagent (0.5 mmol) is added portionwise, at 110°C, to a solution of 9-methoxy-1-ethyl-2,3,4,6,7,12-hexahydroindolo[2,3-a] quinolizin-4-one (300 mg, 1.01 mmol) in anhydrous toluene (15 ml). After refluxing for 30 min. and evaporation of the toluene, the product is chromatographed on silica gel (99/1 chloroform/methanol eluent) and 9-methoxy-1-ethyl-2,3,4,6,7,12-hexahydroindolo [2,3-a]quinolizine-4-thione is thus recovered (60% yield).
**NMR:** ^{**1**}**H** (CDCl₃) : 1.32 (t, 3H); 2.32 (t, 2H), 2.65 (q, 2H); 2.98 (t, 2H); 3.08 (t, 2H); 3.89 (s, 3H); 4.80 (t, 2H); 6.91 (dd 2.4 and 8.7 Hz, 1H); 6.98 (d 2.4 Hz, 1H); 7.34 (d 8.7 Hz, 1H); 8.11 (broad s, 1H)
**Mass spectrum:**
m/z: 312(M+.) (100), 297
**Melting point:** 118°C

### Example 8 : ETDHCARBO7

Formula : C₁₈H₂₂N₂O₂ M = 298,38 g.mol⁻¹

### Structure :

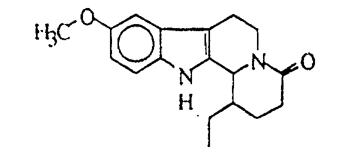

### 9-methoxy-1-ethyl-1,2,3,4,6,7,12,12b-octahydroindolo [2,3-a] quinolizin-4-one

### Preparation:

Sodium bicarbonate (500 mg) and palladium-on-charcoal are successively added to a solution of 9-methoxy-1-ethyl-2,3,4,6,7,12-hexahydroindolo[2,3-a]quinolizin-4-one (500 mg) in ethanol, and the mixture is stirred overnight under a hydrogen atmosphere. After filtration and evaporation of the solvent, the crude product is recrystallized from ethyl acetate. 9-Methoxy-1-ethyl-1,2,3,4,6,7,12,12b-octahydroindolo [2,3-a]quinolizin-4-one is thus recovered (76%).
**NMR:** ^{**1**}**H** (CDCl₃) : 0.79 (t, 3H); 1.13 (2m, 2H); 1.95 (m, 2H) ; 2.20 (m, 1H); 2.45 (m, 2H); 2.77 (m, 2H); 3.87 (s, 3H); 4.88 (s, 1H); 5.09 (m, 1H); 6.78 (dd 2.4 and 8.7 Hz, 1H); 6.92 (d 2.4 Hz, 1H); 7.25 (d 8.7 Hz, 1H); 8.77 (broad s, 1H)
**Melting point:** 207°C

### Example 9 : ETNAPH7

Formula : C₂₀H₂₁NO₂ M = 307,39 g.mol⁻¹

### Structure :

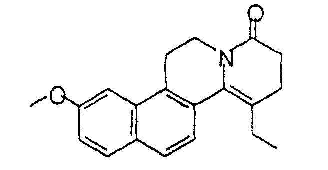

### 11-ethyl-3-methoxy-5,8,9, 10-tetrahydro-6H-benzo[f]pyrido[2,1-a]isoquinolin-8-one

### Preparation:

POCl₃ (4.6 ml) is added to a refluxing solution of the N(2-(7-methoxy-napht-1-yl)ethyl)butanamide (2.27 g, 11.1 mmol) in toluene (80 ml). After 3 h, the toluene is removed under reduced pressure. The residue is taken up in KOH solution (40%). The mixture is then extracted with EtOAc (x 3). After drying the organic phase over MgSO₄, the solvent is removed. The crude reaction product is dissolved in DMF (5 ml) and acrylic acid (0.94 ml, 1.2 eq.) is then added. Diphenylphosphoryl azide (2.7 ml, 1.1 eq.) dissolved in DMF (3 ml) is then added dropwise, followed by triethylamine (3.67 ml, 2.6 eq.). 11-ethyl-3-methoxy-5,8,9,10-tetrahydro-6H-benzo(f)pyrido(2,1-*a*]isoquinolin-8-one (329 mg) is obtained by recrystallization from a 20/80 EtOAc/P.E. mixture).
**NMR:** ^{**1**}**H** (CDCl₃) : 1.17 (t, 3H); 2.38 (m, 4H); 2.58 (t, 2H); 3.16 (t, 2H); 3.82 (t, 2H); 3.95 (s, 3H); 7.17 (dd, 1H); 7.29 (d, 1H); 7.33 (d, 1H); 7.66 (d, 1H); 7.76 (d, 1H)
**Melting point:** 105-107°C

### Example 10 : PHNAPH7

Formula : C₂₄H₂₁NO₂ M = 355,43 g.mol⁻¹

### Structure :

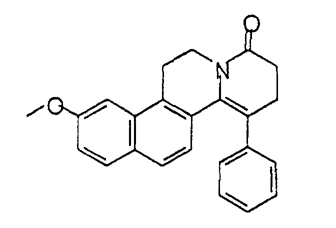

### 3-methoxy-11-phenyl-5,8,9,10-tetrahydro-6H-benzo[f]pyrido [2, 1-a]isoquinolin-8-one

### Preparation :

POCl₃ (1.4 ml) is added to a refluxing solution of the N(2-(7-methoxy-napht-1-yl)ethyl)phenylacetamide (600 mg) in toluene (100 ml). After 3 h, the toluene is removed under reduced pressure. The residue is taken up in KOH solution (40%). The mixture is then extracted with EtOAc (x 3). After drying the organic phase over MgSO₄, the solvent is removed. The crude reaction product is dissolved in DMF (4.5 ml) and acrylic acid (0.15 ml, 1.1 eq.) is then added. Diphenylphosphoryl azide (0.45 ml, 1.1 eq.) dissolved in DMF (1 ml) is then added dropwise, followed by triethylamine (0.55 ml, 2.1 eq.). The 3-methoxy-11-phenyl-5, 8,9,10-tetrahydro-6H-benzo[f]pyrido [2,1-*a*]isoquinolin-8-one is recovered (170 mg, yield = 25%).
**NMR:** ^{**1**}**H** (CDCl₃) : 2.70 (m, 4H): 3.32 (t, 2H) ; 3.88 (t, 2H); 3.94 (s, 3H); 6.74 to 7.60 (10H)
**Melting point:** 152-154°C

### Example 11 : PHCARBO7S

Formula : C₂₂H₂₀NOS M = 360,47 g.mol⁻¹

### Structure :

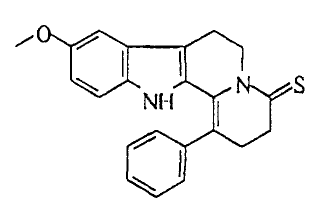

### 9-methoxy-1-phenyl-2,3,4,6,7,12-hexahydroindolo [2,3-a] [beta]quinolizin 4-thione

### Preparation :

Lawesson's reagent (180 mg, 0.47 mmol) is added portionwise, at 110°C, to a solution of 9-methoxy-1-phenyl-2,3,4,6,7,12-hexahydroindolo[2,3-*a*]quinolizin-4-one (164 mg, 0.47 mmol) in anhydrous toluene (10 ml). After refluxing for 30 min. and evaporation of the toluene, the product is chromatographed on silica gel (chloroform eluent) and the 9-methoxy-1-phenyl-2,3,4,6,7,12-hexahydroindolo[2,3-a]quinolizine-4-thione is thus recovered (130 mg, 76% yield).
**NMR:** ^{**1**}**H** (CDCl₃) : 1.32 (t, 3H); 2.32 (t, 2H), 2.65 (q, 2H); 2.98 (t, 2H); 3.08 (t, 2H); 3.89 (s, 3H); 4.80 (t, 2H); 6.91 (dd 2.4 and 8.7 Hz, 1H); 6.98 (d 2.4 Hz, 1H); 7.34 (d 8.7 Hz, 1H); 8.11 (broad s, 1H)
**Melting point:** 180°C

### Example 12 : DEETCARBO7S

Formula : C₁₈H₁₈N₂OS M = 310,41g.mol⁻¹

### Structure :

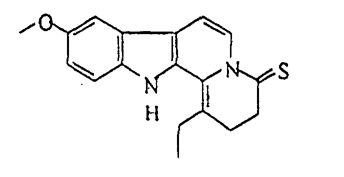

### 9-methoxy-1-ethyl-2,3,4,12-tetrahydroindolo[2,3-a] quinolizin-4-thione

### Preparation :

Potassium tert-butoxide (665 mg, 5.9 mmol) is added to a solution of 9-methoxy-1-ethyl-2,3,4,6,7,12-hexahydroindolo [2,3-*a*]quinolizine-4-thione (500 mg, 1.6 mmol) in DMF (42 ml). After placing the reaction assembly under vacuum, the mixture is stirred under normal oxygen pressure overnight. Water (15 ml) and concentrated hydrochloric acid (3 ml) are then successively added. The solution is stored in a refrigerator for 4 hours. After filtration, 9-methoxy-1-ethyl-2,3,4,12-tetrahydroindolo [2,3-a] quinolizine-4-thione is obtained (150 mg, Y = 30%).
**NMR:** ^{**1**}**H** (CDCl₃) : 1.38 (t, 3H), 2.93 (q, 2H), 3.06 (t, 2H), 3.90 (s, 3H), 5.06 (t, 2H), 7.03 (d+s, 2H), 7.19 (d, 1H), 7.38 (d, 1H), 7.62 (d, 1H), 8.43 (broad s, 1H)
**Mass spectrum:**
m/z: 310 (M+.), 295, 155
**Melting point:** 212°C

### Example 13 : FPHCARBO7

Formula : C₂₂H₁₉N₂O₂F M = 362,40g.mol⁻¹

### Structure :

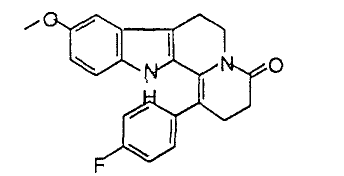

### 9-methoxy-1-(p-fluorophenyl)-2,3,4,6,7,12-hexahydroindolo [2,3-a]quinolizin-4-one

### Preparation :

A Bischler-Napieralski reaction, carried out on N-[2'-(5-methoxy-1H-3-indolyl)ethyl]-2-(*p*-fluorophenyl)acetamide (1.15 g, 3.5 mmol), results in the 1-(parafluorobenzyl)-6-methoxy-3,4-dihydro-2-carboline (1.06 g), which is dissolved directly in anhydrous dimethylformamide (DMF) (10 ml).
This mixture is cooled to 0°C and acrylic acid (0.24 ml, 3.5 mmol), diphenylphosphoryl azide ((PhO)₂P(O)N₃) (0.74 ml, 3.5 mmol) in solution in DMF (2 ml), dropwise, and triethylamine (1 ml, 7.8 mmol) are successively added. After separation on silica gel (chloroform), 9-methoxy-1-(*p*-fluoro-phenyl)-2,3,4,6,7,12 -hexahydroindolo[2, 3-a]quinolizin-4-one is recovered (750 mg, Y = 58%)
**NMR:** ^{**1**}**H** (CDCl₃) : 2,71 (m, 4H), 2,89 (t, 2H), 3,82 (s, 3H), 4.19 (t, 2H), 6.77 (dd, 9 Hz and 3 Hz, 1H), 6.9-7.20 and 7.38 (3m, 7H)
**Mass spectrum:**
m/z: 362 (M+.), 319, 253
**Melting point:** 191°C

### Example 14 : ANCARBO7

Formula : C₂₃H₂₂N₂O₃ M = 374,43g.mol⁻¹

### Structure :

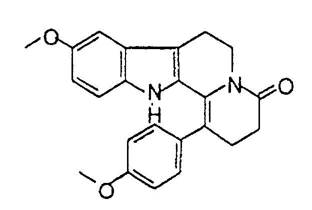

### 9-methoxy-1-(p-methoxyphényl) -2,3,4,6,7, 12-hexahydroindolo [2,3-a]quinolizin-4-one

### Preparation :

A Bischler-Napieralski reaction, carried out on N-[2'-(5-methoxy-1H-3-indolyl)ethyl]-2-(p-methoxyphenyl)acetamide (800 mg, 2.1 mmol), results in the 1-(paramethoxybenzyl)-6-methoxy-3,4-dihydro-2-carboline, which is dissolved directly in anhydrous dimethylformamide (DMF) (10 ml).
This mixture is cooled to 0°C and acrylic acid (0.15 ml, 2.2 mmol), diphenylphosphoryl azide ((PhO)₂P(O)N₃) (0.45 ml, 2 mmol) in solution in DMF (2 ml), dropwise, and triethylamine (0.53 ml, 4 mmol) are successively added. After separation on silica gel (chloroform), 9-methoxy-1-(*p*-methoxyphenyl)-2,3,4,6,7,12-hexahydroindolo[2,3-a]quinolizin-4-one is recovered (213 mg, Y = 27%).
**NMR:** ^{**1**}**H** (CDCl₃) : 2.67 (m, 4H), 2.90 (t, 6 Hz, 2H), 3.83 (s, 3H), 3.91 (s, 3H), 4.20 (t, 6 Hz, 2H), 6.77, 6.88, 7.04 and 7.33 (m, 7H)
**Mass spectrum:**
m/z: 374 (M+. 100), 359, 253, 187
**Melting point:** 154-155°C

### Example 15 : DMACARBO7

Formula : C₂₄H₂₅N₃O₂ M = 387,48g.mol⁻¹

### Structure :

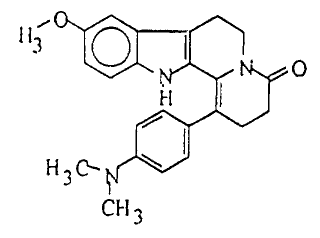

### 9-méthoxy-1-(p-diméthylaminophényl)-2,3,4,6,7,12-hexahydroindolo[2, 3-a]quinolizin-4-one

### Préparation :

A Bischler-Napieralski reaction, carried out on N-[2'-(5-methoxy-1H-3-indolyl)ethyl]-2-(*p*-dimethylaminophenyl)-acetamide, results in the 1-(*para*dimethylaminobenzyl)-6-methoxy-3,4-dihydro-2-carboline, which is dissolved directly in anhydrous dimethylformamide (DMF).
This mixture is cooled to 0°C and acrylic acid, diphenylphosphoryl azide ((PhO)₂P(O)N₃) in solution in DMF, dropwise, and triethylamine are successively added. After separation on silica gel (chloroform), 9-methoxy-1-(*p*-di-methylaminophenyl)-2,3,4,6,7,12-hexahydroindolo[2,3-*a*]-quinolizin-4-one is recovered.
**NMR:** ^{**1**}**H** (CDCl₃) : 2.69 (m, 4H), 2.89 (t, 2H), 3.04 (s, 6H), 3.82 (s, 3H), 4.20 (t, 2H), 6.80 (m, 3H), 6.89 (m, 2H), 7.25 (d, 2H), 7.29 (broad s, 1H)
**Mass spectrum:** m/z: 387 (M+.), 194, 142, 134 (100)

### Example 16 : PYRCARBO7

Formula : C₂₁H₁₉N₃O₂ M = 345, 40 g.mol⁻¹

### Structure :

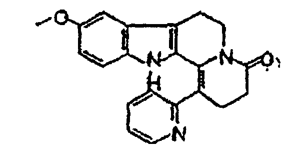

### 9-methoxy-1-(pyrid-2'-yl)-2,3,4,6,7,12-hexahydroindolo [2,3-a]quinolizin-4-one

### Preparation :

A Bischler-Napieralski reaction on the N1-(2-(5-methoxy-1H-3-indolyl)ethyl)-2-(pyrid-2'-yl)-acetamide (1.2g - 3.9 mml) leads to 1-[2-pyridylmethyl)-6-methoxy-3,4-dihydro-2-carboline.
To a solution of 1-(2-pyridylmethyl)-6-methoxy-3,4-dihydro-2-carboline (760mg - 2.6 mmol) in the DMF (9ml), acrylic acid is added (0.18 ml). Diphenylphosphorylazide (0.55 ml) is then added drip in solution in the DMF (3 ml), then the trietylamine (0.75 ml).
After separation on silica gel (Chloroform/methanol), 9-methoxyl-1-(pyrid-2'-yl)-2,3,4,6,7,12-hexahydroindolo[2,3-*a*]quinolizin-4-one (230mg-17.5%) is recovered.
**NMR:** ^{**1**}**H** (CDCl₃) : 2,73 (m, 2H); 2,85 (m, 2H); 2,94 (t, 2H); 3,18 (s, 3H), 4,24 (t, 2H); 6,85 (dd 2,4 et 9Hz, 1H); 6,93 (d 2,4Hz, 1H); 7,10 (d 9Hz, 1H); 7,30(dd, 1H) ; 7,45(d, 1H) ; 7,80 (dd, 1H) ; 8,75 (d, 1H)
**Mass Spectrum:**
m/z : 345(M⁺, 100), 330, 316, 302
**Melting point:** > 260°C

### Example 17 : NPHCARBO7

Formula : C₂₃H₁₉N₃O₄ M = 389,41 g.mol⁻¹

### Structure :

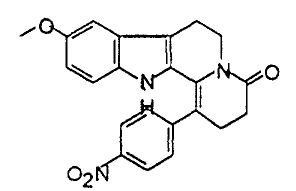

### 9-methoxy-1-p-nitrophenyl-2,3,4,6,7,12-hexahydroindolo[2,3-a] quinolizin-4-one

### Preparation :

A Bischler-Napieralski reaction on the N1-(2-(5-methoxy-1H-3-indolyl)ethyl)-2-*p*-nitrophenyl-acetamide (360mg - lmmol) leads to 1-(*para*nitrobenzyl)-6-methoxy-3,4-dihydro-2-carboline.

To a solution of 1-(*para*nitrobenzyl)-6-methoxy-3,4-dihydro-2-carboline in the DMF (10ml), acrylic acid (0.07ml) is added. Diphenylphosphorylazide (0.21 ml) is then added drip and then triethylamine (0.26ml). After separation on silica gel (eluent AcOEt/EP - 50/50), 9-methoxy-1-*p*-nitrophenyl-2,3,4,6,7,12-hexahydroindolo[2,3-*a*]quinolizin-4-one (163mg-41%) is recovered.
**NMR :** ^{**1**}**H** (CDCl₃) :2,75 (m,4H); 2,92 (t, 2H); 3,82 (s, 3H); 4,17 (t,2H); 6,80 (dd 3 et 9 Hz, 1H) ; 6,90 (d 3 Hz, 1H); 6,92 (d 9Hz, 1H); 7,59 (d, 9Hz, 1H); 8,32 (, 9Hz, 1H).
**Melting point:** 138-140°C

### Example 18 : TOLCARBO7

Formula : C₂₃H₂₂N₂O₂ M = 358,43 g.mol⁻¹

### Structure :

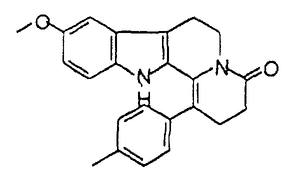

### 9-méthoxy-1-p-tolyl-2,3,4,6,7,12-hexahydroindolo[2,3-a] quinolizin-4-one

### Preparation :

A Bischler-Napieralski reaction on the N1-(2-(5-methoxy-1H-3-indolyl)ethyl)-2-*p*-tolyl-acetamide (2.12mg - 6.6mmol) leads to 1-(*para*methylbenzyl)-6-methoxy-3,4-dihydro-2-carboline.

To a solution of 1-(*para*methylbenzyl)-6-methoxy-3,4-dihydro-2-carboline in the DMF (15ml), acrylic acid (0.46ml) is added. Diphenylphosphorylazide (1.4 ml) is then added drip and then triethylamine (1.75ml). After separation on silica gel (eluent Chloroform/methanol), 9-methoxy-1-*p*-tolyl-2,3,4,6, 7,12-hexahydroindolo[2,3-*a*]quinolizin-4-one is recovered.
**NMR :** ^{**1**}**H** (CDCl₃) : 2,46 (s, 3H) ; 2,73 (m, 4H); 2,88 (t, 2H); 3,82 (s, 3H); 4,20 (t, 2H); 6,75 (dd 2 et 9Hz, 1H); 6,84 (d 9Hz, 1H); 6,88 (d 2Hz, 1H); 7,03 (s large, 1H); 7,30 (m, 4H)
**Melting point:** 198°C

### Example 19 : PHDHCARBO7

Formula: C₂₂H₂₂N₂O₂ M = 346.42g.mol⁻¹

### Structure :

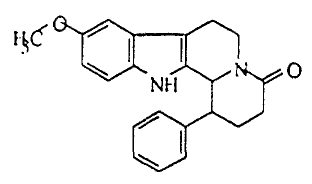

### 9-methoxy-1-phenyl-1,2,3,4,6,7,12,12b-octahydroindolo[2,3-a]quinolizin-4-one

### Preparation :

Sodium bicarbonate (900 mg) and palladium on charcoal are successively added to a solution of 9-methoxy-1-phenyl-2,3,4,6,7,12-hexahydroindolo[2,3-*a*]quinolizin-4-one (2.4 g) in mixture ethyl acetate - ethanol 1/1 (160 mL), and the mixture is stirred overnight under a hydrogen atmosphere.

After filtration and evaporation of the solvent, the crude product is recrystallized from ethyl acetate. 9-methoxy-1-phenyl-1,2,3,4,6,7,12,12b-octahydroindolo[2,3-a]quinolizin-4 -one is thus recovered (2.3 g, yield = 95%).
**NMR :** ^{**1**}**H** (CDCl₃) : 2.05 (m, 1H), 2.28 (m, 1H), 2.47 (m, 2H), 2.74 (m, 1H), 2.97 (m, 2H), 3.81 (m + s, 4H), 5.18 (m, 2H), 6.71 (dd, 2.4 Hz and 9 Hz, 1H), 6.89 (d, 2.4 Hz, 1H), 6.93 (d, 9Hz, 1H),7.25 (m, 5H), 7.5 (broad s, 1H).
**Mass spectrum :**
m/z : 346 (M+.), 242, 200.
**Melting point:** 162°C

### Example 20 : FPHDHCARBO7

Formula: C₂₂H₂₂N₂FO₂ M = 364,41 g mol⁻¹

### Structure :

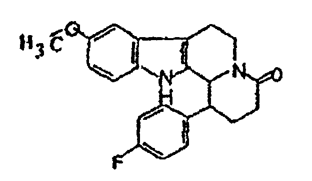

### 9-methoxy-1-(parafluorophenyl)-1,2,3,4,6,7,12,12 boctahydroindolo[2,3-a]quinolizin-4-one

### Preparation :

Sodium bicarbonate (50 mg) and palladium on charcoal are successively added to a solution *of* 9-methoxy-1-(*paraf*luorophenyl)-2,3,4,6,7,12-hexahydroindolo[2,3-*a*] quinolizin-4-one (64 mg) in ethyl acetate, and the mixture is stirred overnight under a hydrogen atmosphere. After filtration and evaporation of the solvent, the crude product is recrystallized from ethyl acetate. 9-methoxy-1-(*para*fluorophenyl)-1,2,3,4,6,7,12,12b-octahydroindolo[2,3-*a*] quinolizin-4-one is thus recovered (21 mg, yield = 33%).
**NMR :** ^{**1**}**H** (CDCl₃) : 2.05 (m, 1H), 2.40 (m, 3H), 2.74 (m, 1H), 2.90 (m, 2H), 3.80 (1s+1m, 4H), 5.19 (m, 2H), 6.71 (dd, 2.4 and 9 Hz, 1H), 6.92 (m, 3H), 6.97 (d, 8.7 Hz, 1H), 7.18 (m, 2H), 7.52 (broad s, 1H).
**Mass spectrum :**
m/z : 364 (M+.), 242, 200.
**Melting point:** 162-166°C

### Example 21 : PHDHNAPH7

Formula: C₂₄H₂₃NO₂ M = 357.45g.mol⁻¹

### Structure :

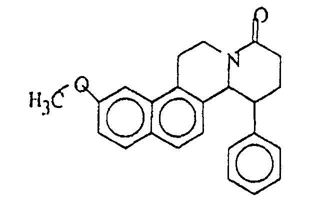

### 3-methoxy-11-phenyl-5,8,9,10,11,11a-hexahydro -6H-benzo[f] pyrido[2,1-a]isoquinolin-8-one

### Preparation :

Sodium bicarbonate (20 mg) and palladium-on charcoal are successively added to a solution of 3-methoxy-11-phenyl-5,8,9,10-tetrahydro-6H-benzo[f]pyrido[2,1-*a*]isoquinolin-8-one (30 mg) in ethyl acetate (15 mL) and the mixture is stirred overnight under a hydrogen atmosphere. After filtration and evaporation of the solvent, the crude product is recrystallized from ethyl acetate. 3-methoxy-11-phenyl-5,8,9,10,11,11a-hexahydro -6H-benzo[f]pyrido[2,1-*a*] isoquinolin-8- one is thus recovered (25mg, yield = 83%).
**NMR :**^{**1**}**H** (CDCl₃) : 2.14 (m, 1H), 2.48 to 3.16 (m, 5H), 3.13 (d, 15.6 Hz, 1H), 3.94 (m + s, 4H), 5.16 (ddd, 12.8 Hz, 5.1 Hz and 2.1 Hz, 1H), 5.28 (d, 4.4 Hz, 1H), 6.89 to 7.13 (m, 8H), 7.55 (d, 8.5Hz, 1H), 7.62 (d, 9.6Hz, 1H).
**Mass spectrum :**
m/z : 357 (M+.), 329, 253, 211
**Melting point:** 175°C

### Example 22 : ETDHNAPH7

Formula: C₂₀H₂₃NO₂ M = 309.40g.mol⁻¹

### Structure :

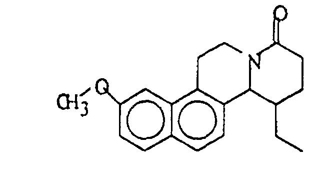

### 11-ethyl-3-methoxy-5,8,9,10,11,11a-hexahydro -6H-benzo[f] pyrido[2,1-a]isoquinolin-8-one

### Preparation :

Sodium bicarbonate (30 mg) and palladium on charcoal are successively added to a solution of 11-ethyl- 3-methoxy-5,8,9,10-tetrahydro-6H-benzo[f]pyrido[2,1-*a*]isoquinolin-8-one (74 mg) in ethyl acetate (25 mL) and the mixture is stirred overnight under a hydrogen atmosphere. After filtration and evaporation of the solvent, the crude product is recrystallized from ethyl acetate. 3-methoxy-11-ethyl-5,8,9,10,11,11a-hexahydro -6H-benzo[f]pyrido[2,1-*a*] isoquinolin-8-one is thus recovered (50 mg, yield = 67%).
**NMR :** ^{**1**}**H** (CDCl₃) : 0.73 (t, 6.8Hz, 3H), 1.08 (m, 2H), 2.05 (m, 2H), 2.30 to 3.05 (m, 5H), 3.16 (d, 16.0 Hz, 1H), 3.96 (s, 3H), 5.02 (broad s, 1H), 5,17 (ddd, 12.4 Hz, 4.4 Hz and 16 Hz, 1H), 7.12 to 7.21 (m, 3H), 7.67 (d, 8.6Hz, 1H), 7.73 (d, 8.6Hz, 1H).
**Mass spectrum :**
m/z : 309 (M+.), 253, 225, 211.
**Melting point:** 206°C

### Example 23 : ANIDHCARBO7

Formula: C₂₂H₂₃N₃O₂ M = 361.45g.mol⁻¹

### Structure :

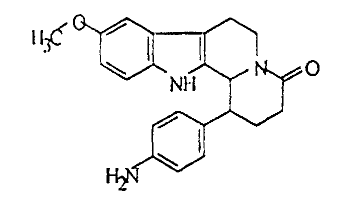

### 9-methoxy-1-paraaminophenyl-1, 2,3,4,6,7,12,12b octahydroindolo[2,3-a]quinolizin-4-one

### Preparation :

Sodium bicarbonate (50 mg) and palladium on charcoal are successively added to a solution of 9-methoxy-1-*para*nitrophenyl-2,3,4,6,7,12-hexahydroindolo[2,3-*a*] quinolizin-4-one (91 mg) in ethyl acetate and the mixture is stirred overnight under a hydrogen atmosphere. After filtration and evaporation of the solvent, the crude product is recrystallized from ethyl acetate. 9-methoxy-1-*para*aminophenyl-1,2,3,4,6,7,12,12b-octahydroindolo[2,3-*a*] quinolizin-4-one is thus recovered (37 mg, yield 44%).
**NMR :** ^{**1**}**H** (CDCl₃) : 1.95 (m, 1H), 2.28 (m, 1H), 2.40 (m, 2H), 2.90 (m, 2H), 3.70 (m, 1H), 3.80 (m, 3H), 4.20 (broad s, 2H), 5.20 (m, 2H), 6.49 (d, 8.3 Hz, 1H), 6.65 (dd, 8.7 Hz, 2.4 Hz, 1H), 6.83 (d, 2.4 Hz, 1H), 6.90 (d, 8.4 Hz, 1H), 6.95 (d, 8.7 Hz, 1H).
**Mass spectrum :**
m/z : 361 (M+.), 242, 200, 181.
**Melting point:** 165-166°C

### Example 24 : PHDHCARBO7S

Formula: C₂₂H₂₂N₂O_{S} M = 362.48g.mol⁻¹

### Structure :

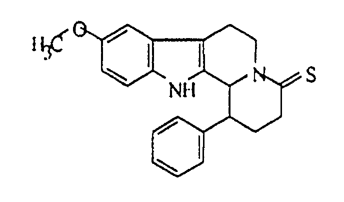

### 9-methoxy-1-phenyl-1,2,3,4,6,7,12,12b- octahydroindolo [2,3-a]quinolizin-4-thione

### Preparation :

To 9-methoxy-1-phenyl-1,2,3,4,6,7,12,12b-octahydroindolo [2,3-*a*]quinolizin-4-one (520 mg) in anhydrous toluene (50mL) we added, at 110°C, Lawesson reactif (640 mg), in small quantities. After refluxing for 30 min and evaporation of toluene, the product is isolated by column chromatography over SiO₂ (Eluent chloroform/methanol, 99/1), we obtained the 9-methoxy-1-phenyl-1,2,3,4,6,7,12,12b-octahydroindolo [2,3-*a*]quinolizine-4-thione (160 mg, yield 30%).
**NMR :** ^{**1**}**H** (CDCl₃) : 1.95 (m, 1H), 2.20 (m, 1H), 2.72 (dd, 12 Hz, j = 2.8 Hz, 1H), 2.97 (m, 3H), 3.41 (m, 1H), 3.80 (m + s, 4H), 5.33 (d, j = 4 Hz, 1H), 5.93 (dd, 5.1 Hz and 1.3 Hz, 1H), 6.71 (dd, 8.7 Hz and 2,4 Hz, 1H), 6.83 (d, 2.4Hz, 1H), 6.94 (d, 8.7 Hz, 1H), 7.20 (m, 5H).
**Melting point:** 242-244°C

### Example 25 : ETNAPH7S

Formula: C₂₀H₂₁NOS M = 323.45g.mol⁻¹

### Structure :

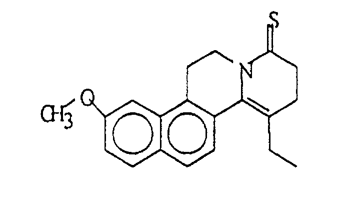

### 11-ethyl-3-methoxy-5,8,9,10-tetrahydro-6H-benzo [f] pyrido [2,1-a]isoquinolin-8-thione

### Preparation :

To 11-ethyl-3-methoxy-5,8,9,10-tetrahydro-6H-benzo [f] pyrido [2,1-*a*] isoquinolin-8-one (45 mg) in anhydrous toluene (10mL) we added, at 110°C, Lawesson reactif (70mmol),in small quantities . After refluxing for 30 min and evaporation of toluene the product is isolated by column chromatography over SiO₂ (Eluent dichloromethane), we obtained the 11-ethyl-3-methoxy-5,8,9,10-tetrahydro-6H-benzo if] pyrido [2,1-*a*] isoquinolin-8-thione (32 mg, yield 70%).
**NMR :** ^{**1**}**H** (CDCl₃) : 1.14 (t, 7.3 Hz, 3H), 2.35 (m, 4H), 3.13 (t, 7.7 Hz, 2H), 3..25 (t, 6 Hz, 2H), 3.99 (s, 3H), 4.27 (t, 6 Hz, 2H), 7.19 (dd, 8.5 and 2.4 Hz, 1H), 7.28 (m, 2H), 7.67 (d, 8.5 Hz, 1H), 7.76 (d, 8.9 Hz, 1H).
**Melting point:** 164-166°C

### Example 26 : PHNAPH7S

Formula: C₂₄H₂₁NOS M = 371.49g.mol⁻¹

### Structure :

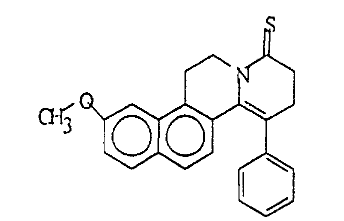

### 3-methoxy-11-phenyl-5,8,9, 10-tetrahydro-6H-benzo [f] pyrido [2,1-a]isoquinolin-8-thione

### Preparation :

To 3-methoxy-11-phenyl-5,8,9,10-tetrahydro-6H-benzo [f] pyrido [2,1-*a*] isoquinolin-8-one (50 mg) in anhydrous toluene (10mL) we added, at 110°C, Lawesson reactif (66 mg),in small quantities . After refluxing for 30 min and evaporation of toluene the product is isolated by column chromatography over SiO₂ (dichloromethane), we obtained the 3-methoxy-11-phenyl-5,8,9,10-tetrahydro-6H-benzo [f] pyrido [2,1-*a*] isoquinolin-8-thione (37mg, yield 74%).
**NMR :** ^{**1**}**H** (CDCl₃) : 2.71 (t, 7.8 Hz, 1H), 3.36 (m, 4H), 3.97 (s, 3H), 4.36 (t, 6.2 Hz, 1H), 6.68 (d, 8.5 Hz, 1H), 7.18 (m, 8H), 7.63 (d, 8.7 Hz, 1H).
**Melting point:** 136°C

### Example 27 : ETDHCARBO7S

Formula : C₁₈H₂₂N₂OS M = 314,44 g.mol⁻¹

### Structure :

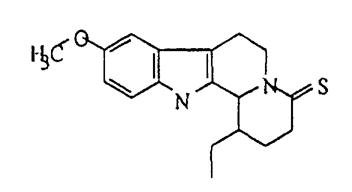

### 1-ethyl -9-methoxy -1,2,3,4,6,7,12,12b-octahydroindolo[2,3-a]quinolizine-4-thione

### Preparation :

To 1-ethyl -9-methoxy -1,2,3,4,6,7,12,12b-octahydroindolo [2,3-*a*]quinolizine-4-one (262 mg) in anhydrous toluene (20 mL) we added, at 110°c, Lawesson reactif (208 mg) in small quantities. After refluxing for 30 min and evaporation of toluene, the product is isolated by column chromatography over SiO₂ (Eluent chloroform/methanol, 99/1) we obtained the 1-ethyl -9-methoxy -1,2,3,4,6,7, 12,12b-octahydroindolo[2,3-a]quinolizine-4-thione (30 mg, yield 11.5%).
**NMR:** ^{**1**}**H** (CDCl₃) : 0.81 (t, 3H), 1.06 (m, 2H), 1.90 (m, 2H) 2.30 (m,1H), 2.80 (m, 5H), 3.85 (s, 3H), 4.94 (s, 1H), 6.08 (m, 1H), 6.87 (dd, 2.4 and 8.7Hz, 1H), 6.96 (d, 2.4Hz, 1H), 7.26 (d, 8.7Hz, 1H), 7.82 (s large, 1H).
**Melting point:** 124°C

### Example 28 : CO2ETDHCARBO7

Formula : C₁₉H₂₂N₂O₄ M = 342, 39 g.mol⁻¹

### Structure :

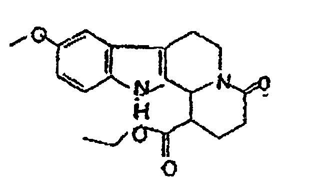

### 1-carbethoxy-9-methoxy-1,2,3,4,6,7,12,12b-octahydroindolo [2,3-a]quinolizin-4-one

### Preparation :

Sodium bicarbonate (10 mg) and palladium on charcoal are successively added to a solution of 1-carbethoxy-9-methoxy-2,3,4,6,7,12-hexahydroindolo[2,3-*a*]quinolizin-4-one (50 mg) in ethyl acetate, and the mixture is stirred overnight under hydrogen atmosphere. After filtration and evaporation of the solvent, the crude product is recrystallized from ethyl acetate. 1-carbethoxy-9-methoxy-1,2,3,4,6,7,12,12b-octahydroindolo[2,3-*a*]quinolizin-4-one is thus recovered (30 mg, yield = 60%).
**NMR :** ^{**1**}**H** (CDCl₃) : 1.02 (t, 3H), 2.15 (m, 2H), 2.85 (m, 8H), 3.35 (m, 1H), 3.85 (s, 3H), 4.05 (t, 2H), 5.06 (m, 2H), 6.82 (dd, 8.7 and 2.4Hz, 1H), 6.92 (d, 2.4Hz, 1H), 7.21 (d, 8.7Hz, 1H), 8.24 (broad s, 1H).
**Mass spectrum :**
m/z : 342 (M+.). 286, 269, 240, 199.
**Melting point:** 213°C

### Example 29 : TOLDHCARBO7

Formula: C₂₃H₂₄N₂O₂ M = 360.46g.mol⁻¹

### Structure :

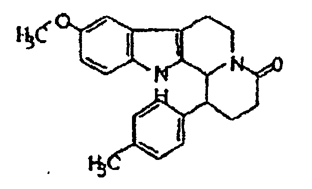

### 9-methoxy-1-paratolyl-1,2,3,4,6,7,12,12b-octahydroindolo [2,3-a]quinolizin-4-one

### Preparation :

Sodium bicarbonate (50 mg) and palladium on charcoal are successively added to a solution of 9-methoxy-1-paratolyl-2,3,4,6,7,12-hexahydroindolo[2,3-*a*]quinolizin-4-one (55 mg) in ethyl acetate and the mixture is stirred overnight under a hydrogen atmosphere. After filtration and evaporation of the solvent, the crude product is recrystallized from ethyl acetate, 9-methoxy-1-paratolyl-1,2,3,4,6,7,12,12b-Octahydroindolo[2,3-*a*]quinolizin-4-one is thus recovered (32 mg, yield 58%).
**NMR :** ^{**1**}**H** (CDCl₃) : 2.04 (m, 1H), 2.23 (m, 1H), 2.29 (s, 3H), 2.46 (m, 2H), 2.74 (m, 1H), 2.94 (m, 2H), 3.70 (m, 1H), 3.80 (s, 3H), 5.20 (m, 1H), 5.24 (d, 1H), 6.71 (dd, 2.4Hz and 8,7Hz, 1H), 6.87 (d, 2.4Hz, 1H), 6.93 (d, 8.7Hz, 1H), 7.08 (d, 8.0Hz, 2H), 7.16 (d, 8.0Hz, 2H), 7.2 (1H, NH).

### Example 30 : ANDHCARBO7

Formula: C₂₃H₂₄N₂O₃ M = 376.46g.mol⁻¹

### Structure :

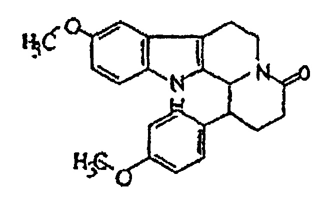

### 9-methoxy-1-paraanisyl-1,2,3,4,6,7,12,12b-octahydroindolo [2,3-a]quinolizin-4-one

### Preparation :

Sodium bicarbonate (50 mg) and palladium on charcoal are successively added to a solution of 9-methoxy-1-paraanisyl-2,3,4,6,7,12-hexahydroindolo[2,3-*a*]quinolizin-4-one (43 mg) in ethyl acetate and the mixture is stirred overnight under a hydrogen atmosphere. After filtration and evaporation of the solvent, the crude product is recrystallized from ethyl acetate, 9-methoxy-1-paraanisyl-1,2,3,4,6,7,12,12b-octahydroindolo[2,3-*a*]quinolizin-4-one is thus recovered (24 mg, yield 56%).
**NMR :**^{**1**}**H** (CDCl₃) : 2.05 (m, 1H), 2.25 (m, 1H), 2.46 (m, 2H), 2.74 (d, 1H), 2.54 (m, 2H), 3.68 (m, 1H), 3.75(s, 3H), 3.80 (s, 3H), 5.14 (m, 1H), 5.23 (d, 5.5Hz, 1H), 6.72 (dd, 2.4 and 8.7Hz, 1H), 6.82 (d, 8.7Hz, 2H), 6.87 (d, 2.4Hz, 1H), 6.96 (d, 8.7Hz, 1H), 7,17(d, 8.7Hz, 2H).
**Melting point:** 242°C

### Example 31 : DIETCARBO7

Formula : C₂₀H₂₆N₂O₂ M = 326.43g.mol⁻¹

### Structure :

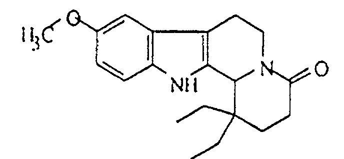

### 1,1-diethyl -9-methoxy -1,2, 3,4,6,7, 12,12b-octahydroindolo [2,3-a]quinolizine-4-one

### Preparation :

5-methoxytryptamine (494 mg - 2,59 mmol) and ethyl (-4-ethyl -4-formyl) caproate (522 mg - 2,61 mmol) are mixed in commercial toluene (27 mL) in a 50 mL flask.
The medium is then heated to the toluene reflux during 2 hours. After cooling, the toluene is evaporated under reduced pressure and acetic acid (1 mL) is added.
The medium is then heated to the acetic acid reflux during 2 hours. After cooling, water (25 mL) is added and a solid precipitates. This solid is diluted with ethyl acetate and washed with water. The resulting organic phase is dried over magnesium sulfate and the solvent is evaporated under reduced pressure.
After separation on silica gel (chloroform/methanol-97.5/2.5 eluent), 1,1-diethyl -9-methoxy -1,2,3,4,6,7, 12,12b-octahydroindolo[2,3-*a*]quinolizine-4-one-is obtained (200 mg , yield 23%).
**NMR:** ^{**1**}**H** (CDCl₃) : 0.73 (t, 7.5Hz, 3H), 1.02 (q, 7.5Hz, 1H), 1.18 (t, 7.5Hz, 3H) 1.50 (q 7.5Hz, 1H), 1.60 (m, 1H), 1.80 (m, 4H), 2.49 (m, 1H), 2.76 (m, 3H), 3.85 (s, 3H), 4.83 (s, 1H), 5.17 (m, 1H), 6.84 (dd, 2.3 and 8.7Hz, 1H), 6.85 (d, 2.3Hz, 1H), 7.24 (d, 8.7Hz, 1H), 7.77 (s broad , 1H).
**Melting point:** 229°C

### Example 32 : ETCARBO7TL

Formula: C₂₁H₂₄N₂O₄ M = 368.43g.mol⁻¹

### Structure :

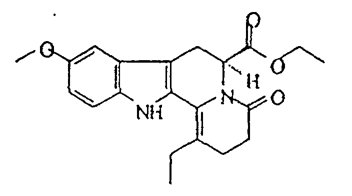

### (6S) -6-carbethoxy-9-methoxy-1-ethyl-2,3,4,6,7,12-hexahydroindolo[2,3-a]quinolizin-4-one

### Preparation :

A Bischler-Napieralski reaction on N1-(butyryl)-5-methoxy-(L)-tryptophan-ethyl ester (2.1 g) lead to the (3*S*)-1-propyl-3-carbethoxy-6-methoxy-3,4-dihydro-2-carboline (1.5 g). To a solution of (3*S*)-1-propyl-3-carbethoxy-6-methoxy-3,4-dihydro-2-carboline (1.5 g) in DMF (20 mL) was added dropwise successively acrylic acid (0.35 mL), diphenylphosphoryl azide (1 mL) and triethylamine (2 mL). After separation on silica gel (eluent - ethyl acetate/petroleum ether - 30/70), (6*S*)-6-carbethoxy-9-methoxy-1-ethyl-2,3,4,6,7,12-hexahydroindolo[2,3-*a*]quinolizin-4-one is recovered (400 mg - 17%)
**NMR :** ^{**1**}**H** (CDCl₃) :1.1(t, 3H), 1.25(t, 3H), 2.3(m, 1H), 2.62(m,5H), 3.08(dd, 6 and 16 Hz, 1H), 3.53(d, 16Hz, 1H), 3.85(s, 3H), 4.03(m, 2H), 6.00(d, 6Hz, 1H), 6.85(dd, 2.3 and 8.7 Hz, 1H), 6.94(d, 2.3Hz, 1H), 7.24(d, 8.7Hz, 1H), 8.13 ( *s* broad, 1 H)
**Melting point:** 192°C

## Claims

1. Derivatives general formula Ia (Ia' and Ia"): 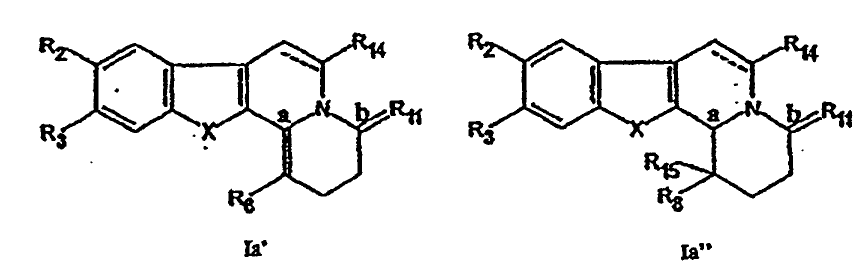 in which
R₂ represents a hydroxyl or (C₁-C₆) alkoxy radical,
R₃ represents an hydrogen atom or an alkyl radical,
R₈ being an alkyl radical, optionally substitued by alkyl, halide, amino, alkyloxy groups, alkyloxycarbonyl, a phenyl or a pyridyl group optionally substitued by alkyl, halide, nitro, dialkylamino, alkyloxy group, alkyloxycarbonyl, alkylamino,
X represents preferably a divalent radical of formula 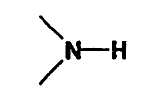 or
―HC=CH―
R₁₁ represents an oxygen atom or sulfur atom,
R₁₄ and R₁₅ represent, independently of each other, a hydrogen atom, a (C₁-C₆) alkyl, a (C₃-C₆) cycloalkyl, halo(C₁-C₆) alkyl, perhalo (C₁-C₆) alkyl, aryl, aralkyl, (C₁-C₆) alkoxy, (C₃-C₆) cycloalkoxy, mono or polyhaloalkoxy, aryloxy, aralkyloxy, hydroxyalkyl, alkyloxyalkyl, (C₁-C₆) alkylthio, (C₃-C₆) cycloalkylthio, mono or polyhaloalkylthio, arylthio, aralkylthio, formate, (C₁-C₆) alkylcarbonyloxy, a (C₃-C₆) cycloalkylcarbonyloxy, halo(C₁-C₃) alkylcarbonyloxy, arylcarbonyloxy, aralkylcarbonyloxy, dialkylaminoalkyl, dialkylamino, diarylamino, diaralkylamino, optionally substitued carboxamide, fornamide, a (C₁-C₆) alkylcarbonylamino, a (C₃-C₆) cycloalkylcarbonylamino, halo (C₁-C₆) alkylcarbonylamino, perhalo (C₁-C₆) alkylcarbonylamino, (C₃-C₆) arylcarbonylamino, (C₁-C₆) aralkylcarbonylamino, formyl, a (C₁-C₆) alkylcarbonyl, a (C₃-C₆) cycloalkylcarbonyl, halo (C₁-C₆) alkylcarbonyl, perhalo (C₁-C₆) alkylcarbonyl, arylcarbonyl, aralkylcarbonyl, carboxyl, (C₁-C₆) alkoxycarbonyl, (C₃-C₆) cycloalkoxycarbonyl, haloalkoxycarbonyl, aryloxycarbonyl, aralkyloxycarbonyl, a (C₁-C₆) alkylsulfonyl, a (C₃-C₆) cycloalkylsulfonyl, halo (C₁-C₆) alkylsulfonyl, perhalo (C₁-C₆) alkylsulfonyl, arylsulfonyl, aralkylsulfonyl, cyano or nitro radical.

2. Derivatives according to claim 1 wherein R₂ is a methoxy radical.

3. Derivatives according to one of the preceding claims wherein R₃ is a methyl radical.

4. Derivatives according to one of the preceding claims wherein R₈ is an ethyl, hexyl, isopropyl radical, a phenyl, fluorophenyl, methoxyphenyl, aminophenyl, dimethylaminophenyl, nitrophenyl, p. methylphenyl radical, an ethoxycarbonyl radical, a pyridine radical.

5. Derivatives according to one of the preceding claims, selected from the following derivatives :
1. 9-methoxy-1-ethyl-2,3,4,6,7,12-hexahydroindolo[2,3-a]quinolizin-4-one,
2. 9-methoxy-1-hexyl-2,3,4,6,7,12-hexahydroindolo[2,3-a]quinolizin-4-one,
3. 9-methoxy-1-isopropyl-2,3,4,6,7,12-hexahydroindolo[2,3-a]quinolizin-4-one,
4. 9-methoxy-1-phenyl-2,3,4,6,7,12-hexahydroindolo[2,3-a]quinolizin-4-one,
5. 1-carbethoxy-9-methoxy-2,3,4,6,7,12-hexahydroindolo[2,3-a]quinolizin-4-one,
6. 9-methoxy-1,10-diethyl-2,3,4,6,7,12-hexahydroindolo[2,3-a]quinolizin-4-one,
7. 9-methoxy-1-ethyl-2,3,4,6,7,12-hexahydroindolo[2,3-a]quinolizine-4-thione,
8. 9-methoxy-1-ethyl-1,2,3,4,6,7,12,12b-octahydroindolo[2,3-a] quinolizin-4-one,
9. 11-ethyl-3-methoxy-5,8,9,10-tetrahydro-6H-Benzo[f]pyrido[2,1-a] isoquinolin-8-one,
10. 3-methoxy-11-phenyl-5,8,9,10-tetrahydro-6H-benzo[f] pyrido[2,1-a]isoquinolin-8-one,
11. 9-methoxy-1-phenyl-2,3,4,6,7,12-hexahydroindolo[2,3-a][beta]-quinolizine-4-thione,
12. 9-methoxy-1-ethyl-2,3,4,12-tetrahydroindolo [2,3-a] quinolizin-4-thione,
13. 9-methoxy-1-(p-fluorophenyl)-2,3,4,6,7,12-hexahydroindolo [2,3-a] quinolizin-4-one,
14. 9-methoxy-1-(p-methoxyphenyl)-2,3,4,6,7,12-hexahydroindolo[2,3-a] quinolizin-4-one,
15. 9-méthoxy-1-(p-diméthylaminophényl)-2,3,4,6,7,12-hexahydroindolo [2,3-a] quinolizin-4-one,
16. 9-methoxy-1-(pyrid-2'-yl)-2,3,4,6,7,12-hexahydroindolo [2,3-a]quinolizin-4-one,
17. 9-methoxy-1-p-nitrophenyl-2,3,4,6,7,12-hexahydroindolo[2,3-a] quinolizin-4-one
18. 9-methoxy-1-p-tolyl-2,3,4,6,7,12-hexahydroindolo[2,3-a] quinolizin-4-one,
19. 9-methoxy-1-phenyl-1,2,3,4,6,7,12,12b-octahydroindolo [2,3-a]quinolizin-4-one,
20. 9-methoxy-1-(parafluorophenyl)-1,2,3,4,6,7,12,12b-octahydroindolo [2,3-a]quinolizin-4-one
21. 3-methoxy-11-phenyl-5,8,9,10,11,11a-hexahydro-6H-benzo[f]pyrido[2,1-a]isoquinolin-8-one
22. 11-ethyl-3-methoxy-5,8,9,10,11,11a-hexahydro-6H-benzo[f]pyrido[2,1-a]isoquinolin-8-one
23. 9-methoxy-1-paraaminophenyl-1,2,3,4,6,7,12,12b-octahydroindolo[2,3-a]quinolizin-4-one
24. 9-methoxy-1-phenyl-1,2,3,4,6,7,12,12b-octahydroindolo [2,3-a]quinolizin-4-thione
25. 11-ethyl-3-methoxy-5,8,9,10-tetrahydro-6H-benzo [f] pyrido [2,1-a] isoquinolin-8-thione
26. 3-methoxy-11-phenyl-5,8,9,10-tetrahydro-6H-benzo [f] pyrido [2,1-a] isoquinolin-8-thione
27. 1-ethyl -9-methoxy -1,2,3,4,6,7,12,12b-octahydroindolo[2,3-a]quinolizine-4-thione
28.1-carbethoxy-9-methoxy-1,2,3,4,6,7,12,12b-octahydroindolo[2,3-a]quinolizin-4-one
29. 9-methoxy-1-paratolyl-1,2,3,4,6,7,12,12b-octahydroindolo[2,3-a]quinolizin-4-one
30. 9-methoxy-1-paraanisyl-1,2,3,4,6,7,12,12b-octahydroindolo [2,3-a]quinolizin-4-one
31. 1,1-diethyl-9-methoxy-1,2,3,4,6,7,12,12b,-octahydroindolo[2,3-a]quinolizine-4-one
32. (6*S*)-6-carbethoxy-9-methoxy-1-ethyl-2,3,4,6,7,12-hexahydroindolo[2,3-a]quinolizin-4-one

6. Derivatives according to one of the preceding claims, racemic mixtures thereof, pure enantiomers thereof, the mixtures thereof in all proportions, and the therapeutically acceptable salts thereof.

7. Process for the preparation of derivatives according to one of the preceding claims.

8. Process according to claim 7 for the preparation of derivatives of general formula 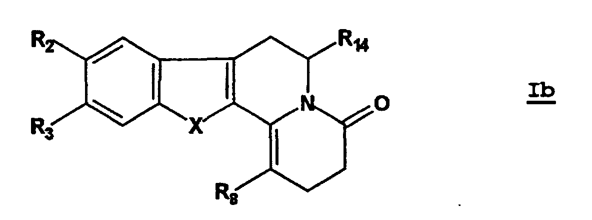 by directly reacting the compound of general formula **IIa.** 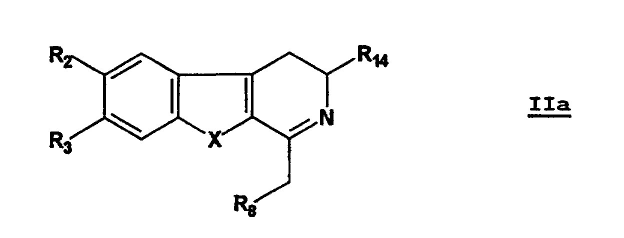 with a carboxylic acid in the presence or not of diphenylphosphorylazide or with acrylonitrile.

9. Process for the preparation of derivatives of general formula **IIa** as an intermediate, by carrying out a Bischler-Napieralski reaction on the compounds of general formula **IIIa**. 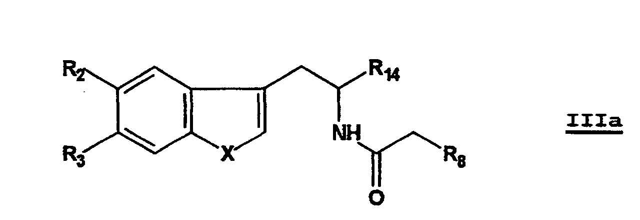 with phosphorus pentoxide (P₂O₅) or with phosphorus oxychloride (POCl₃) in a suitable solvent.

10. Process for the preparation of derivatives of general formula **IIa** by permanganic oxidation of the derivatives of general formula **IIb**

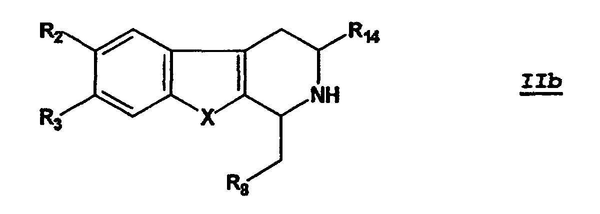

11. Process for the preparation of derivatives of general formula **IIb,** by carrying out a Pictet-Spengler reaction on the derivatives of general formula **IIIb.** 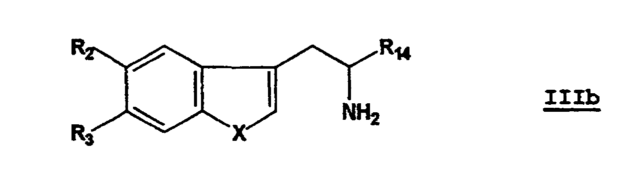 with compounds of formula R₈-CH₂-COH or chemical equivalents thereof under reducing conditions.

12. Process according to claim 7 for the preparation of derivatives of general formula **Ic** 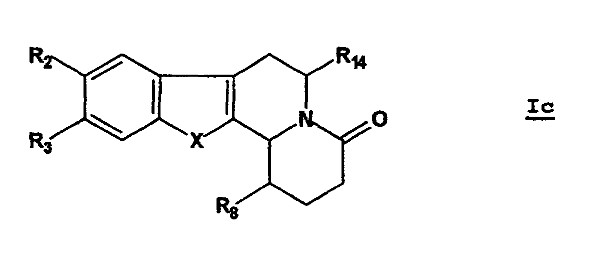 by catalytic reduction by hydrogen with Palladium on Carbon of compound of general formula **Ib**

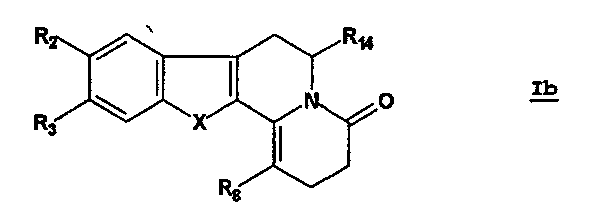

13. Process according to claim 7 for the preparation of derivatives of general **Id** 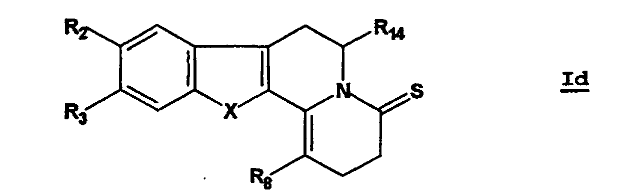 by reaction of the Lawesson's reagent or by P₂S₄ on the compounds of general formula **Ib**

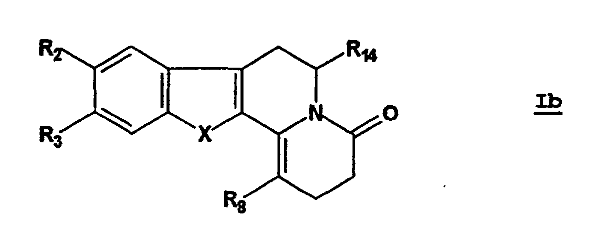

14. Process according to claim 7 for the preparation of derivatives of general **Ic** 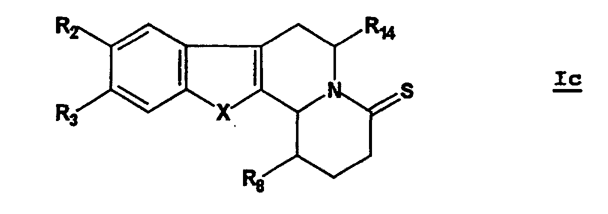 by reaction of the Lawesson's reagent or by P₂S₄ on the compound of general formula **Ic**

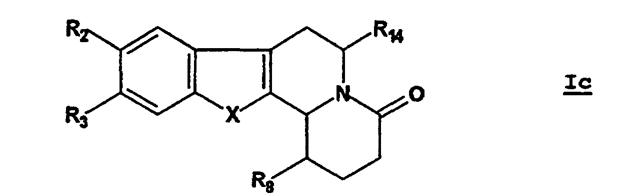

15. Process according to claim 7 for the preparation of derivatives of general **If** 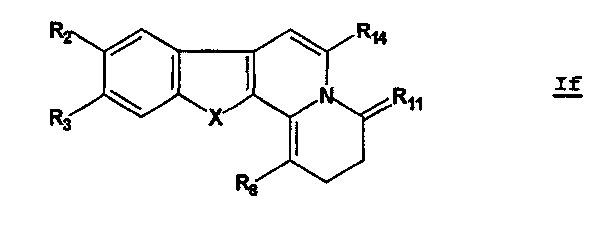 By reaction with an oxidant (such O₂ in alkaline medium) on the compound of general formula **Ib** 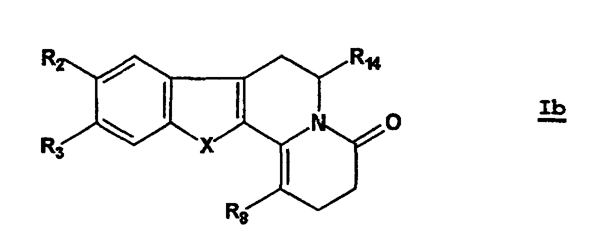 or **Id**

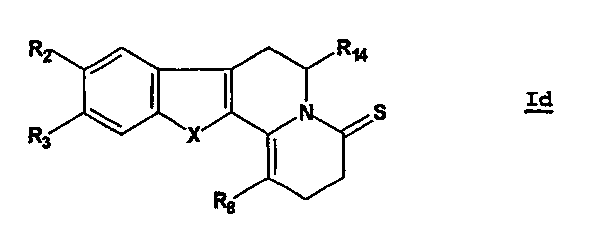

16. Process for the preparation of derivatives of general formula **Ia,** wherein R₈ and R₁₅ are identical by carrying out a reaction on the derivatives of general formula **IIIb**. 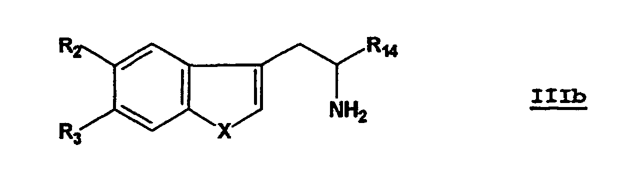 with compounds of formula EtO-CO-C(R₈) (R₁₅)-COH or chemical equivalents thereof under reducing conditions.

17. A medical product **characterized in that** it contains a derivative according to one of the claims 1 to 6 or obtained by a process according to one of the claims 7 to 16.

18. A medical product according to claim 17 having at least one activity selected in the myorelaxing (decontracting), hypnotic, sedative, contraceptive and analgesic activities, an activity for the treatment of diseases associated with disorders of melatonin activity, for the treatment of depression and psychiatric disorders, in particular stress, anxiety, depression, insomnia, schizophrenia, psychoses and epilepsy, for the treatment of sleeping disorders associated with travelling ("jet lag"), neurodegenerative diseases of the central nervous system, for the treatment of cancers.

## Patentansprüche

1. Derivate der allgemeinen Formel Ia (Ia' und Ia''): 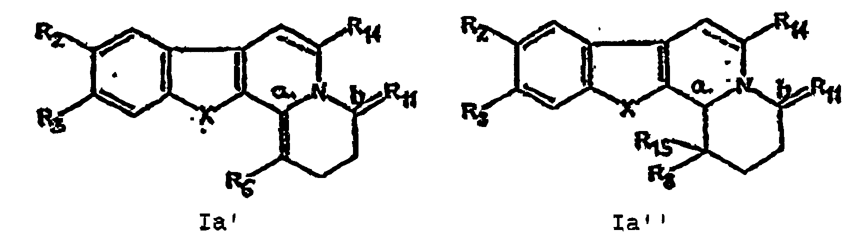 worin
R₂ ein Hydroxyl- oder (C₁-C₆)-Alkoxyradikal,
R₃ ein Wasserstoffatom oder ein Alkylradikal,
R₈ ein Alkylradikal, gegebenenfalls substituiert durch eine Alkyl-, Halid-, Amino-, Alkyloxygruppe, Alkyloxycarbonyl, ein Phenyl oder eine Pyridylgruppe, gegebenenfalls substituiert durch eine Alkyl-, Halid-, Nitro-, Dialkylamino-, Alkyloxygruppe, Alkyloxycarbonyl, Alkylamino,
X bevorzugt ein divalentes Radikal der Formel 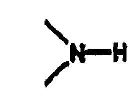 oder
―HC=CH―
R₁₁ ein Sauerstoffatom oder ein Schwefelatom,
R₁₄ und R₁₅ unabhängig voneinander ein Wasserstoffatom, ein (C₁-C₆) -Alkyl, ein (C₁-C₆)-Cycloalkyl, Halo(C₁-C₆) -alkyl, Perhalo(C₁-C₆)-alkyl, Aryl, Aralkyl, (C₁-C₆)-alkoxy, (C₃-C₆)-Cycloalkoxy, Mono- oder Polyhaloalkoxy, Aryloxy, Aralkyloxy, Hydroxyalkyl, Alkyloxyalkyl, (C₁-C₆) -Alkylthio, (C₃-C₆)-Cycloalkylthio, Mono- oder Polyhaloalkylthio, Arylthio, Aralkylthio, Format, (C₁-C₆)-Alkylcarbonyloxy, ein (C₃-C₆)-Cycloalkylcarbonyloxy, Halo(C₁-C₃)-alkylcarbonyloxy, Arylcarbonyloxy, Aralkylcarbonyloxy, Dialkylaminoalkyl, Dialkylamino, Diarylamin, Diaralkylamino, gegebenenfalls substituiertes Carboxamid, Formamid, ein (C₁-C₆)-Alkylcarbonylamino, ein (C₃-C₆)-Cycloalkylcarbonylamino, Halo(C₁-C₆)-alkylcarbonylamino, Perhalo(C₁-C₆)-alkylcarbonylamino, (C₃-C₆)-Arylcarbonylamino, (C₂-C₆)-Aralkylcarbonylamino, Formyl, ein (C₁-C₆)-Alkylcarbonyl, ein (C₃-C₆)-Cycloalkylcarbonyl, Halo(C₁-C₆)-alkylcarbonyl, Perhalo(C₁-C₆)-alkylcarbonyl, Axylcarbonyl, Aralkylcarbonyl, Carboxyl, (C₁-C₆)-Alkoxycarbonyl, (C₃-C₆) -Cycloalkoxycarbonyl, Haloalkoxycarbonyl, Aryloxycarbonyl; Aralkyloxycarbonyl, ein (C₁-C₆)-Alkylsulfonyl, ein (C₃-C₆)-Cycloalkylsulfonyl, Halo(C₁-C₆)-alkylsulfonyl, Perhalo(C₁-C₆)-alkylsulfonyl, Arylsulfonyl, Aralkylsulfonyl, Cyano- oder Nitroradikal darstellen.

2. Derivate gemäß Anspruch 1, worin R₂ ein Methoxyradikal ist.

3. Derivate gemäß einem der vorhergehenden Ansprüche, worin R₃ ein Methylradikal ist.

4. Derivate gemäß einem der vorhergehenden Ansprüche, worin R₅ ein Ethyl-, Hexyl-, Isopropylradikal, éin Phenyl, Fluorphenyl, Methoxyphenyl, Aminophenyl, Dimethylaminophenyl, Nitrophenyl, p.Methylphenylradikal, ein Ethoxycarbonylradikal oder ein Pyridinradikal ist.

5. Derivate gemäß einem der vorhergehenden Ansprüche, ausgewählt aus folgenden Derivaten:
1. 9-Methoxy-1-ethyl-2,3,4,6,7,12-hexahydroindolo[2,3-a]quinolizin-4-on,
2. 9-Methoxy-1-hexyl-2,3,4,6,7,12-hexahydroindolo[2,3-a]quinolizin-4-on,
3. 9-Methoxy-1-isopropyl-2,3,4,6,7,12-hexahydroindolo[2,3-a]quinolizin-4-on,
4. 9-Methoxy-1-phenyl-2,3,4,6,7,12-hexahydroindolo[2,3-a]quinolizin-4-on,
5. 1-Carbethoxy-9-methoxy-2,3,4,6,7,12-hexahydroindolo[2,3-a]quinolizin-4-on,
6. 9-Methoxy-1,10-diethyl-2,3,4,6,7,12-hexahydroindolo[2,3-a]quinolizin-4-on,
7. 9-Methoxy-1-ethyl-2,3,4,6,7,12-hexahydroindolo[2,3-a]quinolizin-4-thion,
8. 9-Methoxy-1-ethyl-1,2,3,4,6,7,12,12b-octahydroindolo[2,3-a]quinolizin-4-on,
9. 11-Ethyl-3-methoxy-5,8,9,10-tetrahydro-6H-benzo[f]pyrido[2,1-a]isoquinolin-8-on,
10. 3-Methoxy-11-phenyl-5,8,9,10-tetrahydro-6H-benzo[f]pyrido[2,1-a]isoquinolin-8-on,
11. 9-Methoxy-1-phenyl-2,3,4,6,7,12-hexahydroindolo[2,3-a][beta]-quinolizin-4-thion,
12. 9-Methoxy-1-ethyl-2,3,4,12-tetrahydroindolo[2,3-a]quinolizin-4-thion,
13. 9-Methoxy-1-(p-fluorphenyl)-2,3,4,6,7,12-hexahydroindolo[2,3-a]quinolizin-4-on,
14. 9-Methoxy-1-(p-methoxyphenyl)-2,3,4,6,7,12-hexahydroindolo[2,3-a]quinolizin-4-on,
15. 9-Methoxy-1-(p-dimethylaminophenyl)-2,3,4,6,7,12-hexahydroindolo[2,3-a]quinolizin-4-on,
16. 9-Methoxy-1-(pyrid-2'-yl)-2,3,4,6,7,12-hexahydroindolo[2,3-a]quinolizin-4-on,
17. 9-Methoxy-1-p-nitrophenyl-2,3,4,6,7,12-hexahydroindolo[2,3-a]quinolizin-4-on,
18. 9-Methoxy-1-p-tolyl-2,3,4,6,7,12-hexahydroindolo[2,3-a]quinolizin-4-on,
19. 9-Methoxy-1-phenyl-1,2,3,4,6,7,12,12b-octahydroindolo[2,3-a]quinolizin-4-on,
20. 9-Methoxy-1-(*para*fluorphenyl)-1,2,3,4,6,7,12,12b-octahydroindolo[2,3-a]quinolizin-4-on,
21. 3-Methoxy-11-phenyl-5,8,9,10,11,11a-hexahydro-6H-benzo[f]pyrido[2,1-a]isoquinolin-8-on,
22. 11-Ethyl-3-methoxy-5,8,9,10,11,11a-hexahydro-6H-benzo[f]pyrido[2,1-*a*]isoquinolin-8-on,
23, 9-Methoxy-1-*para*aminophenyl-1,2,3,4,6,7,12,12b-octahydroindolo[2,3-*a*]quinolizin-4-on,
24. 9-Methoxy-1-phenyl-1,2,3,4,6,7,12,12b-octahydroindolo[2,3-a]quinolizin-4-thion,
25. 11-Ethyl-3-methoxy-5,8,9,10-tetrahydro-6H-benzo[f]pyrido[2,1-*a*]isoquinolizin-8-thion,
26. 3-Methoxy-11-phenyl-5,8,9,10-tetrahydro-6H-benzo[f]pyrido[2,1-*a*]isoquinolin-8-thion,
27. 1-Ethyl-9-methoxy-1,2,3,4,6,7,12,12b-octahydroindolo[2,3-a]quinolizin-4-thion,
28. 1-Carbethoxy-9-methoxy-1,2,3,4,6,7,12,12b-octahydroindolo[2,3-*a*]quinolizin-4-on,
29. 9-Methoxy-1-paratolyl-1,2,3,4,6,7,12,12b-octahydroindolo[2,3-a]quinolizin-4-on,
30. 9-Methoxy-1-paraanisyl-1,2,3,4,6,7,12,12b-octahydroindolo[2,3-a]quinolizin-4-on,
31. 1,1-Diethyl-9-methoxy-1,2,3,4,6,7,12,12b-octahydroindolo[2,3-a]quinolizin-4-on,
32. (6S)-6-carbethoxy-9-methoxy-1-ethyl-2,3,4,6,7,12-hexahydroindolo[2,3-a]quinolizin-4-on.

6. Derivate gemäß einem der vorhergehenden Ansprüche, ihre racemischen Mischungen, ihre reinen Enantiomeren, deren Mischungen in allen verhältnissen und ihre therapeutisch geeigneten Salze.

7. Verfahren zur Herstellung von Derivaten gemäß einem der vorhergehenden Ansprüche.

8. Verfahren gemäß Anspruch 7 zur Herstellung von Derivaten der allgemeinen Formel 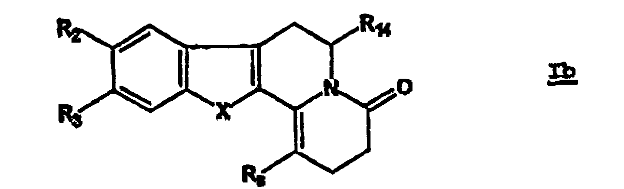 durch direktes Umsetzen der Verbindung der allgemeinen Formel IIa 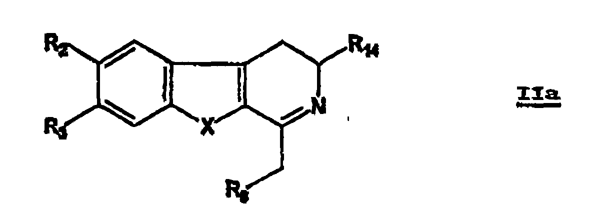 mit einer Carbonsäure in Gegenwart oder Abwesenheit von Diphenylphosphorylazid oder Acrylonitril.

9. Verfahren zur Herstellung von Derivaten der allgemeinen Formel IIa als Zwischenprodukt, in dem eine Bischler-Napieralski-Reaktion an den Verbindungen der allgemeinen Formel IIIa 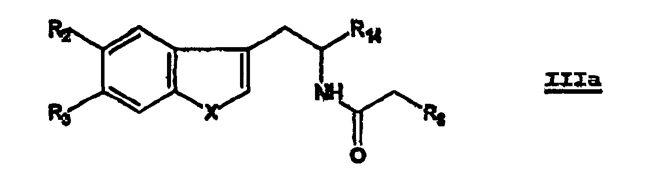 mit Phosphor(III)-pentoxid (P₂O₅) oder mit Phosphor(III)-oxychlorid (POCL₃) in einem geeigneten Lösungsmittel durchgeführt wird.

10. Verfahren zur Herstellung von Derivaten der allgemeinen Formel IIa durch Permanganoxidation der Derivate gemäß der allgemeinen Formel IIb

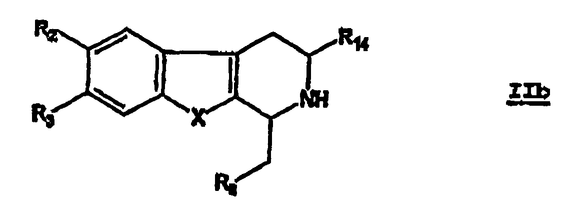

11. Verfahren zur Herstellung von Derivaten der allgemeinen Formel IIb, in dem eine Pictet-Spengler-Reaktion an den Derivaten der allgemeinen Formel IIIb 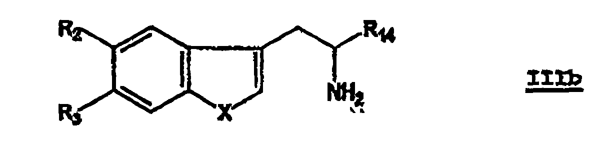 mit Verbindungen der Formel R₆-CH₂-COH oder deren chemischen Äquivalenten unter reduzierenden Bedingungen durchgeführt wird.

12. Verfahren gemäß Anspruch 7 zur Herstellung von Derivaten der allgemeinen Formel Ic 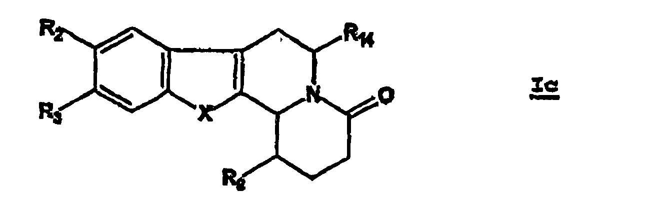 in dem die Verbindung der allgemeinen Formel Ib 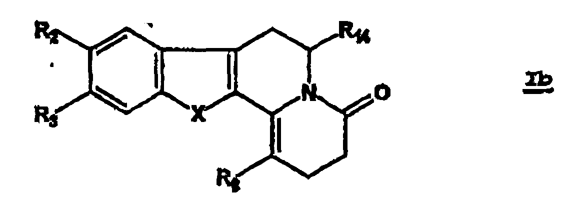 durch Wasserstoff mit Palladium auf Kohlenstoff katalytisch reduziert wird.

13. Verfahren gemäß Anspruch 7 zur Herstellung von Derivaten der allgemeinen Formel Id 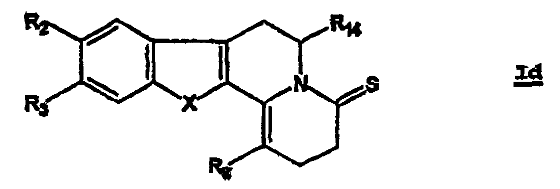 in dem die Verbindungen der allgemeinen Formel Ib 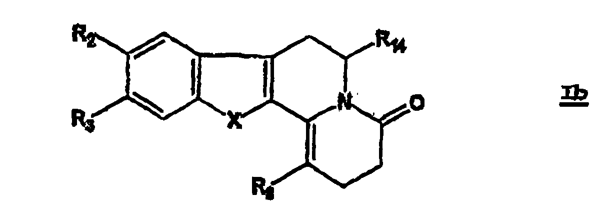 mit dem Lawesson-Reagenz oder mit P₂S₄ umgesetzt werden.

14. Verfahren gemäß Anspruch 7 zur Herstellung von Derivaten der allgemeinen Formel Ic 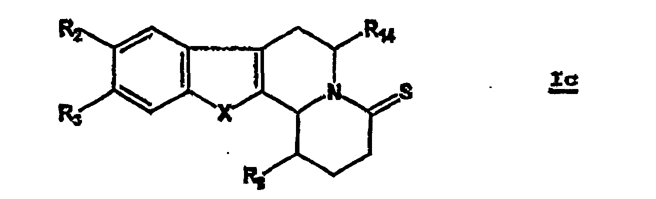 in dem die Verbindung der allgemeinen Formel Ic 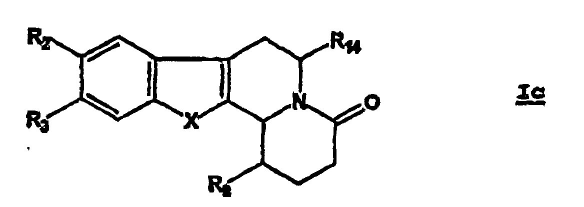 mit dem Lawesson-Reagenz oder mit P₂S₄ umgesetzt wird.

15. Verfahren gemäß Anspruch 7 zur Herstellung von Derivaten der allgemeinen Formel If 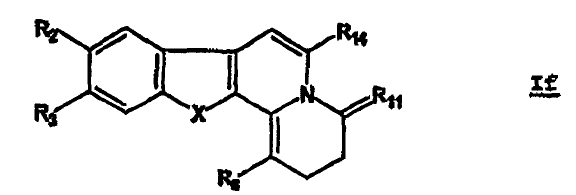 in dem die Verbindung der allgemeinen Formel Ib 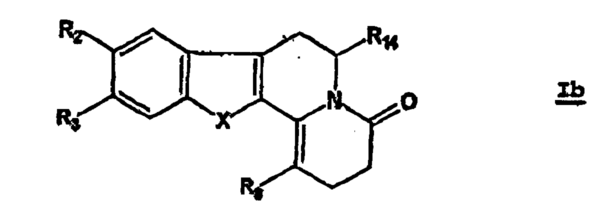 oder Id 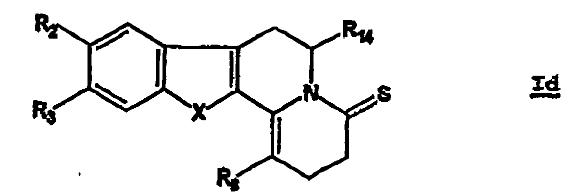 mit einem Oxidationsmittel (wie O₂ in alkalischem Medium) umgesetzt wird.

16. Verfahren zur Herstellung der Derivate der allgemeinen Formel Ia, worin R₈ und R₁₅ identisch sind, in dem die Derivate der allgemeinen Formel IIIb 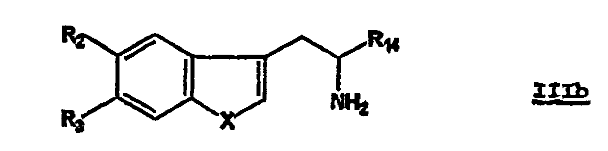 mit Verbindungen der Formel EtO-CO-C (R₈) (R₁₅)-COH oder deren chemischen Äquivalenten unter reduzierenden Bedingungen umgesetzt werden.

17. Medizinisches Produkt, **dadurch gekennzeichnet, dass** es ein Derivat gemäß einem der Ansprüche 1 bis 6 oder ein durch ein Verfahren gemäß einem der Ansprüche 7 bis 16 erhaltenes Derivat enthält.

18. Medizinisches Produkt gemäß Anspruch 17 mit Myorelaxations- (Dekontraktions-)wirkung, hypnotischer Wirkung, sedativer Wirkung, kontrazeptiver und analgetischer Wirkung, einer Wirkung zur Behandlung von Krankheiten, die mit Störungen der Melantonin-Aktivität in Verbindung stehen, zur Behandlung von Depressionen und psychiatrischen Störungen, insbesondere Stress, Angst, Depressionen, Insomnie, Schizophrenie, Psychosen und Epilepsie, zur Behandlung von Schlafstörungen in Verbindung mit Reisen ("Jet Lag"), neurodegenerativen Erkrankungen des zentralen Nervensystems und/oder zur Behandlung von Krebserkrankungen.

## Revendications

1. Dérivés de formule générale la (Ia' et Ia"): 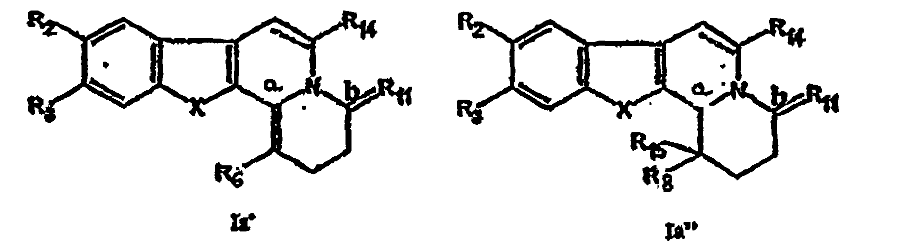 dans laquelle
R₂ représente un radical hydroxyle ou alcoxy en C₁-C₆,
R₃ représente un atome d'hydrogène ou un radical alkyle,
R₄ étant un radical alkyle, éventuellement substitué par des groupes alkyle, halogénure, amino, alkyloxy, alkyloxycarbonyle, un groupe phényle ou un groupe pyridyle éventuellement substitué par un groupe alkyle, halogénure, nitro, dialkylamino, alkyloxy, alkyloxycarbonyle, alkylamino,
X représente de préférence un radical divalent de formule
〉N-H ou-HC=CH-
R₁₁ représente un atome d'oxygène ou un atome de soufre,
R₁₄ et R₁₅ représentent, indépendamment l'un de l'autre, un atome d'hydrogène ou un radical alkyle en C₁-C₆, cycloalkyle en C₃-C₆, halogénoalkyle en C₁-C₆, perhalogénoalkyle en C₁-C₆, aryle, aralkyle, alcoxy en C₁-C₆, cycloalcoxy en C₃-C₆, mono ou polyhalogénoalcoxy, aryloxy, aralkyloxy, hydroxyalkyle, alkyloxyalkyle, alkylthio en C₁-C₆, cycloalkylthio en C₃-C₆, mono or polyhalogénoalkylthio, arylthio, aralkylthio, formate, alkyl(en C₁-C₆)carbonyloxy, cycloalkyl(en C₃-C₆)-carbonyloxy, halogénoalkyl(en C₁-C₃)carbonyloxy, arylcarbonyloxy, aralkylcarbonyloxy, dialkylaminoalkyle, dialkylamino, diarylamino, diaralkylamino, carboxamide éventuellement substitué, formamide, un radical alkyl(en C₁-C₆)carbonylamino, un radical cycloalkyl(en C₃-C₆)-carbonylamino, halogénoalkyl(en C₁-C₆)carbonylamino, perhalogénoalkyl(en C₁-C₆)carbonylamino, aryl(en C₃-C₆)carbonylamino, aralkyl(en C₁-C₆)carbonylamino, formyle, un radical alkyl(en C₁-C₆)carbonyle, un radical cycloalkyl(en C₃-C₆)carbonyle, halogénoalkyl(en C₁-C₆)carbonyle, perhalogénoalkyl(en C₁-C₆)carbonyle, arylcarbonyle, aralkylcarbonyle, carboxyle, alcoxy(en C₁-C₆)carbonyle, cycloalcoxy(en C₃-C₆)carbonyle, halogénoalcoxycarbonyle, aryloxycarbonyle, aralkyloxycarbonyle, un radical alkyl(en C₁-C₆)sulfonyle, un radical cycloalkyl(en C₃-C₆)sulfonyle, halogénoalkyl(en C₁-C₆)sulfonyle, perhalogénoalkyl(en C₁-C₆)sulfonyle, arylsulfonyle, aralkylsulfonyle, cyano ou nitro.

2. Dérivés selon la revendication 1 dans lesquels R₂ est un radical méthoxy.

3. Dérivés selon l'une des revendications précédentes dans lesquels R₃ est un radical méthyle.

4. Dérivés selon l'une des revendications précédentes dans lesquels R₈ est un radical éthyle, hexyle, isopropyle, un radical phényle, fluorophényle, méthoxyphényle, aminophényle, diméthylaminophényle, nitrophényle, p-méthylphényle, un radical éthoxycarbonyle, un radical pyridine.

5. Dérivés selon l'une des revendications précédentes, choisis parmi les dérivés suivants:
1. la 9-méthoxy-1-éthyl-2,3,4,6,7,12-hexahydroindolo[2,3-a]quinolizin-4-one,
2. la 9-méthoxy-1-hexyl-2,3,4,6,7,12-hexahydroindolo[2,3-a]-quinolizin-4-one,
3. la 9-méthoxy-1-isopropyl-2,3,4,6,7,12-hexahydroindolo[2,3-a]-quinolizin-4-one,
4. la 9-méthoxy-1-phényl-2,3,4,6,7,12-hexahydroindolo[2,3-a]-quinolizin-4-one,
5. la 1-carbéthoxy-9-méthoxy-2,3,4,6,7,12-hexahydroindolo[2,3-a]-quinolizin-4-one,
6. la 9-méthoxy-1,10-diéthyl-2,3,4,6,7,12-hexahydxoindolo[2,3-a]-quinolizin-4-one,
7. la 9-méthoxy-1-éthyl-2,3,4,6,7,12-hexahydroindolo[2,3-a]-quinolizine-4-thione,
8. la 9-méthoxy-1-éthyl-1,2,3,4,6,7,12,12b-octahydroindolo[2,3-a]-quinolizin-4-one,
9. la 11-éthyl-3-méthoxy-5,8,9,10-tétrahydro-6H-benzo[f]pyrido-[2,1-a]isoquinoléin-8-one,
10. la 3-méthoxy-11-phényl-5,8,9,10-tétrahydro-6H-benzo[f]pyrido[2,1-a]isoquinoléin-8-one,
11. la 9-méthoxy-1-phényle-2,3,4,6,7,12-hexahydroindolo[2,3-a][bêta]-quinolizine-4-thione,
12. la 9-méthoxy-1-éthyl-2,3,4,12-tétrahydroindolo[2,3-a]quinolizine-4-thione,
13. la 9-méthoxy-1-(p-fluorophényl)-2,3,4,6,7,12-hexahydroindolo[2,3-a]quinolizin-4-one,
14. la 9-méthoxy-1-(p-méthoxyphényl)-2,3,4,6,7,12-hexahydroindolo-[2,3-a]quinolizin-4-one,
15. la 9-méthoxy-1-(p-diméthylaminophényl)-2,3,4,6,7,12-hexahydroindolo[2,3-a]quinolizin-4-one,
16. la 9-méthoxy-1-(pyrid-2'-yl)-2,3,4,6,7,12-hexahydroindolo[2,3-a]-quinolizin-4-one,
17. la 9-méthoxy-1-p-nitrophényl-2,3,4,6,7,12-hexahydroindolo[2,3-a]-quinolizin-4-one
18. la 9-méthoxy-1-p-tolyl-2,3,4,6,7,12-hexahydroindolo(2,3-a]-quinolizin-4-one,
19. la 9-méthoxy-1-phényle-1,2,3,4,6,7,12,12b-octahydroindolo[2,3-a]-quinolizin-4-one,
20. la 9-méthoxy-1-(para-fluorophényl)-1,2,3,4,6,7,12,12b-octahydroindolo[2,3-a]quinolizin-4-one
21. la 3-méthoxy-11-phényl-5,8,9,10,11,11a-hexahydro-6H-benzo[f]-pyrido[2,1-a] isoquinoléin-8-one
22. la 11-éthyl-3-méthoxy-5,8,9,10,11,11a-hexahydro-6H-benzo[f]-pyrido[2,1-a]isoquinoléin-8-one
23. la 9-méthoxy-1-para-aminophényl-1,2,3,4,6,7,12,12b-octahydroindolo-[2,3-a]quinolizin-4-one
24. la 9-méthoxy-1-phényl-1,2,3,4,6,7,12,12b-octahydroindolo[2,3-a]-quinolizine-4-thione
25. la 11-éthyl-3-méthoxy-5,8,9,10-tétrahydro-6H-benzo[f]pyrido-[2,1-a]isoquinoléine-8-thione.
26. la 3-méthoxy-11-phényle-5,8,9,10-tétrahydro-6H-benzo[f]pyrido-[2,1-a]isoquinoléine-8-thione.
27. la 1-éthyl-9-méthoxy-1,2,3,4,6,7,12,12b-octahydroindolo[2,3-a]-quinolizine-4-thione.
28. la 1-carbéthoxy-9-méthoxy-1,2,3,4,6,7,12,12b-octahydroindalo[2,3-a]quinolizin-4-one.
29. la 9-méthoxy-1-para-tolyl-1,2,3,4,6,7,12,12b-octahydroindolo-[2,3-a]quinolixin-4-one.
30. la 9-méthoxy-1-para-anisyl-1,2,3,4,6,7,12,12b-octahydroindolo[2,3-a]quinolizin-4-one.
31. la 1,1-diéthyl-9-méthoxy-1,2,3,4,6,7,12,12b-octahydroindolo[2,3-a]-quinolizin-4-one.
32 la (6S)-6-carbéthoxy-9-méthoxy-1-éthyl-2,3,4,6,7,12-hexahydroindolo[2,3-a]quinolizin-4-one.

6. Dérivés selon l'une des revendications précédentes, leurs mélanges racémiques, leurs énantiomères purs, leurs mélanges en toutes proportions, et leurs sels acceptables en thérapeutique.

7. Procédé de préparation de dérivés selon l'une des revendications précédentes.

8. Procédé selon la revendication 7 pour la préparation de dérivés de formule générale 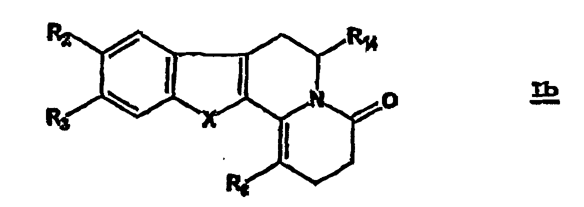 qui consiste à faire réagir directement le composé de formule générale IIa 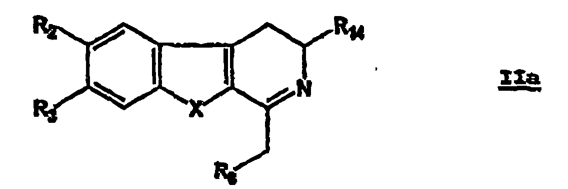 avec un acide carboxylique en présence ou en l'absence d'azoture de diphénylphosphoryle ou avec de l'acrylonitrile.

9. Procédé de préparation de dérivés de formule générale IIa comme intermédiaire, qui consiste à soumettre à une réaction de Bischler-Napieralski les composés de formule générale IIIa 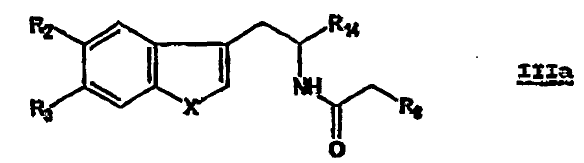 avec du pentoxyde de phosphore (P₂O₅) ou avec de l'oxychlorure de phosphore (POCl₃) dans un solvant approprié.

10. Procédé de préparation de dérivés de formule générale IIa qui consiste à soumettre à une oxydation au permanganate les dérivés de formule générale IIb

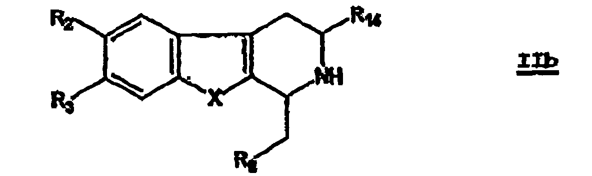

11. Procédé de préparation de dérivés de formule générale IIa qui consiste à soumettre à une réaction de Pictet-Spengler les dérivés de formule générale IIIb 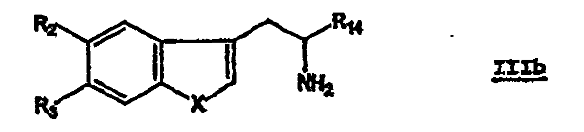 avec des composés de formule R₈-CH₂-COH ou leurs équivalents chimiques, dans des conditions réductrices.

12. Procédé selon la revendication 7 pour la préparation de dérivés de formule générale Ic 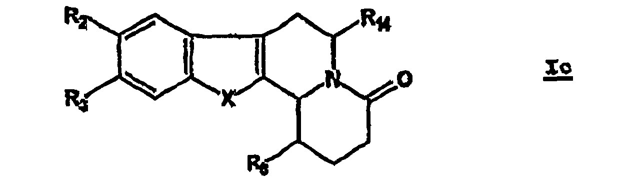 qui consiste à soumettre à une réduction catalytique par l'hydrogène, avec du palladium sur charbon, le composé de formule générale Ib

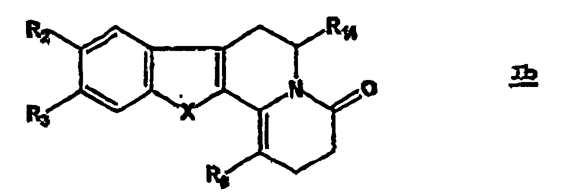

13. Procédé selon la revendication 7 pour la préparation de dérivés de formule générale Id 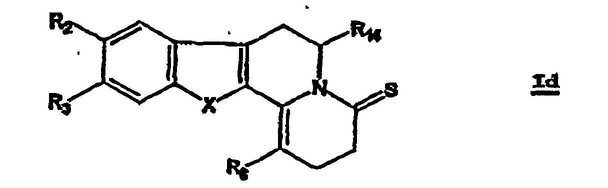 qui consiste à faire réagir le réactif de Lawesson ou P₂S₄ sur les composés de formule générale Ib

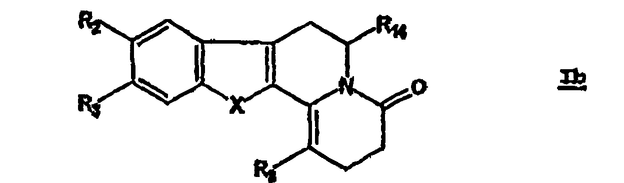

14. Procédé selon la revendication 7 pour la préparation de dérivés de formule générale Ic 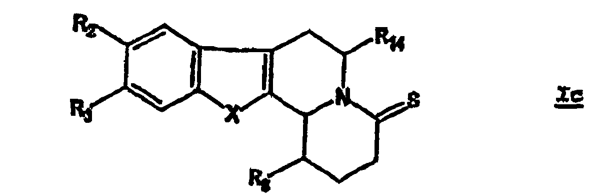 qui consiste à faire réagir le réactif de Lawesson ou P₂S₄ sur le composé de formule générale Ic

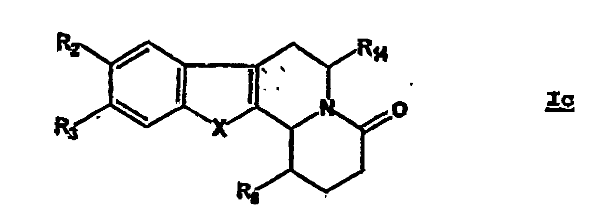

15. Procédé selon la revendication 7 pour la préparation de dérivés de formule générale If 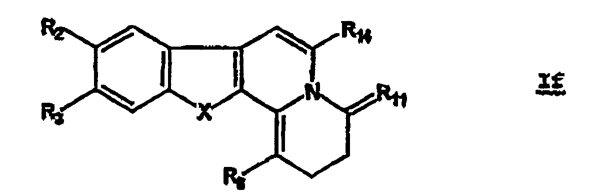 qui consiste à faire réagir avec un oxydant (tel que O₂ dans un milieu alcalin) le composé de formule générale Ib 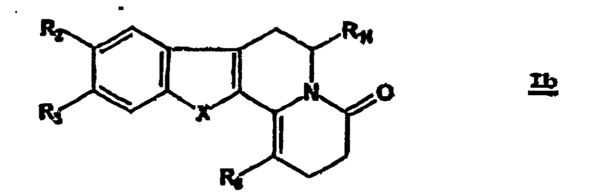 ou Id

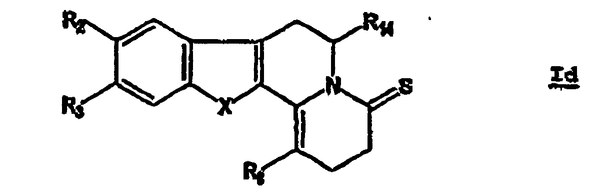

16. Procédé de préparation de dérivés de formule générale Ia, dans lequel R₈ et R₁₅ sont identiques, qui consiste à faire réagir les dérivés de formule générale IIIb 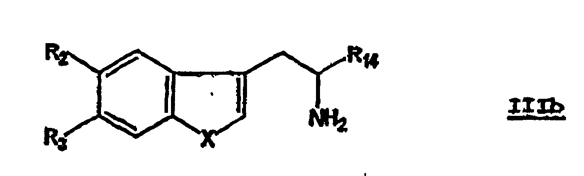 avec des composés de formule EtO-CO-C(R₈)(R₁₅)-COH, ou leurs équivalents chimiques, dans des conditions réductrices.

17. Produit médical **caractérisé en ce qu'**il contient un dérivé selon l'une des revendications 1 à 6 ou obtenu par un procédé selon l'une des revendications 7 à 16.

18. Produit médical selon la revendication 17 ayant au moins une activité choisie parmi les activités myorelaxante (décontractante), hypnotique, sédative, contraceptive et analgésique, une activité pour le traitement de maladies associées à des troubles de l'activité de la mélatonine, pour le traitement de la dépression et des troubles psychiatriques, en particulier le stress, l'anxiété, la dépression, l'insomnie, la schizophrénie, les psychoses et l'épilepsie, pour le traitement des troubles du sommeil associés aux voyages (dus au "décalage horaire"), des maladies neurodégénératives du système nerveux central, pour le traitement de cancers.
